# EUROPÄISCHE PATENTANMELDUNG

(11) **EP 4 285 987 A1**
(43) Veröffentlichungstag der Anmeldung: **06.12.2023**
(21) Anmeldenummer: 23172478.2
(22) Anmeldetag: 10.05.2023
(51) Int. Cl.: A61N 5/06

(54) **SYSTEM ZUR DURCHFÜHRUNG EINER TRANSKUTANEN PHOTODYNAMISCHEN THERAPIE (PDT) IN EINEM ORGAN ODER ORGANSEGMENT EINES ORGANISCHEN KÖRPERS**

(30) Priorität: 03.06.2022 DE 102022205706
(71) Anmelder: Richard Wolf GmbH, 75438 Knittlingen (DE)
(72) Erfinder: Weber, Bernd Claus, 76228 Karlsruhe (DE); Prestel, Stephan, 76287 Rheinstetten (DE); Rieckert, Giacomo, 75438 Knittlingen (DE)
(74) Vertreter: Patentanwälte Vollmann Hemmer Lindfeld Partnerschaft mbB

(57) **Zusammenfassung**

Die vorliegende Offenbarung betrifft ein System (1) zur Durchführung einer transkutanen photodynamischen Therapie (PDT) in einem Organ (3) oder Organsegment eines organischen Körpers (5), wobei das System (1)
- eine Mehrzahl von Lichtapplikatoren (9),
- eine Versorgungseinheit zum Versorgen der Lichtapplikatoren (9) mit Licht und/oder Strom, und
- eine in einer definierten Position zum organischen Körper (5) platzierbare Platzierungsschablone (39) zum definierten Ausrichten, Positionieren und Fixieren der Lichtapplikatoren (9) aufweist, und
- ein Lagerpunktelement (100),

wobei die Lichtapplikatoren (9) jeweils einen nadelartigen Einführabschnitt (15) zum transkutanen Einstechen entlang einer Einstichachse (E) in das Organ (3) oder Organsegment, am distalen Ende (13) des Einführabschnitts (15) eine lichtemittierende Applikatorspitze (19) und mindestens einen definierten Fixierpunkt (43) in einem proximalen Abstand d von der Applikatorspitze (19) aufweisen,
wobei der Abstand d für jeden der Lichtapplikatoren (9) identisch ist, wobei das Lagerpunktelement (100) für jeden der Lichtapplikatoren (9) einen mit der Einstichachse (E) koaxial positionierten oder positionierbaren Lagerpunkt (93) zum lateralen Lagern des jeweiligen Lichtapplikators (9) definiert, wobei ein distaler Abstand f des Lagerpunkts (93) von der Platzierungsschablone (39) einstellbar ist.

## Beschreibung

Die vorliegende Offenbarung betrifft ein System zur Durchführung einer transkutanen photodynamischen Therapie (PDT) in einem Organ oder Organsegment eines organischen Körpers.

Die PDT ist eine bekannte medizinische Therapie von pathologischem Gewebe eines Patienten. Dem Patienten wird dazu ein Photosensibilisator bzw. Markerstoff verabreicht, der sich selektiv an dem zu therapierenden pathologischen Gewebe anreichert. Bei der PDT wird dann Licht mittels eines Lichtapplikators oder mehrerer Lichtapplikatoren unmittelbar auf oder sogar in das pathologische Gewebe appliziert, um lichtinduziert die Bildung von Sauerstoffradikalen mittels des örtlich begrenzt angereicherten Photosensibilisators bzw. Markerstoffs zu fördern und dadurch das pathologische Gewebe, wie etwa einen Tumor, zu zerstören. Typischerweise wird dazu Laserlicht in einen Lichtleiter eingekoppelt und zum Gewebe geleitet. Wenn das pathologische Gewebe flächig auf einer äußeren Oberfläche des Körpers, z.B. der Haut, oder einer inneren Oberfläche, z.B. der Speiseröhreninnenfläche oder Darmwandungen, angeordnet ist, dann kann das Therapielicht relativ einfach ausgekoppelt und auf die pathologische Gewebefläche gestrahlt werden. Erstreckt sich das pathologische Gewebe allerdings über ein Volumen in einem inneren Organ oder Organsegment, so kann wegen der begrenzten Eindringtiefe des Lichts in das Gewebe nicht immer ein Tumor von "außen" effektiv bestrahlt werden. In diesem Fall ist die PDT besonders effektiv, wenn das Licht vom Inneren des pathologischen Gewebevolumens bestrahlt wird. Dazu muss der Lichtapplikator oder die Lichtapplikatoren in das pathologische Gewebe eingestochen werden. Dies wird auch als interstitielle (durch innere Oberflächen) und/oder transkutane (durch die Haut) PDT bezeichnet. Die US 6,048,359 beschreibt beispielsweise ein System zur Durchführung einer transkutanen PDT, um ein inneres Volumen im Körper eines Patienten zu bestrahlen. Dabei wird eine Mehrzahl von nadelartigen Lichtapplikatoren parallel durch die Haut in den Körper gestochen, wobei jeder Lichtapplikator über seine Länge verteilt laterale Lichtauskopplungen aufweist, um so ein Volumen im Körper zu bestrahlen.

Nachteilig an der bekannten Lösung ist, dass die Lichtapplikatoren erstens relativ dick ausgestaltet werden müssen und zweitens relativ komplex und teuer sind, sodass sie erstens, insbesondere in der Summe, relativ patientenbelastend sind und zweitens nicht als Einwegartikel zum einmaligen Gebrauch realisierbar sind. Bei dünneren Lichtapplikatoren besteht das Risiko, dass sie sich beim Einstich in die Haut verbiegen können und somit die Applikatorspitze nicht exakt genug im Körper platziert werden kann.

Daraus ergibt sich die Aufgabe, ein kostengünstigeres System zur Durchführung einer transkutanen PDT mit dünneren und damit patientenschonenderen Lichtapplikatoren bereitzustellen, wobei das System eine exakte Platzierung der Applikatorspitzen im Körper und dadurch eine lückenlose Bestrahlung des Organs oder Organsegments erlauben soll.

Die vorliegende Offenbarung betrifft in dieser Hinsicht eine Weiterentwicklung der zukünftig noch zu veröffentlichenden DE 10 2021 211 328 A1 und DE 102021 211 331 A1.

Gemäß einem ersten Aspekt der vorliegenden Offenbarung wird zur Lösung dieses Problems ein System zur Durchführung einer transkutanen PDT in einem Organ oder Organsegment eines organischen Körpers bereitgestellt, bei dem das System eine Mehrzahl von individuell manuell platzierbaren Lichtapplikatoren, eine Versorgungseinheit zum Versorgen der Lichtapplikatoren mit Licht und/oder Strom, eine in einer definierten Position zum organischen Körper platzierbare Platzierungsschablone zum definierten Ausrichten, Positionieren und Fixieren der einzelnen Lichtapplikatoren in bzw. an der Platzierungsschablone und damit schließlich zur definierten Platzierung der lichtemittierenden Applikatorspitzen im Organ oder Organsegment, und ein Lagerpunktelement aufweist. Dafür weisen die einzeln platzierbaren Lichtapplikatoren jeweils einen nadelartigen Einführabschnitt zum transkutanen Einstechen entlang einer Einstichachse in das Organ oder Organsegment, am distalen Ende des Einführabschnitts eine lichtemittierende Applikatorspitze und mindestens einen definierten Fixierpunkt in einem proximalen Abstand d von der lichtemittierenden Applikatorspitze auf. Dabei ist der Abstand d für jeden der einzelnen Lichtapplikatoren identisch, wobei das Lagerpunktelement für jeden der einzelnen Lichtapplikatoren einen mit der Einstichachse koaxial positionierten oder positionierbaren Lagerpunkt zum lateralen Lagern des jeweiligen Lichtapplikators definiert, wobei ein distaler Abstand f des Lagerpunkts von der Platzierungsschablone einstellbar ist.

Durch das im einstellbaren Abstand f von der Platzierungsschablone angeordnete Lagerpunktelement kann das Risiko einer Durchbiegung der einzelnen Lichtapplikatoren beim Einstich wesentlich reduziert werden. Man könnte auch sagen, dass die einzelnen Lichtapplikatoren durch das Lagerpunktelement weniger biegesteif sein müssen, also dünner und günstiger konstruiert werden können. Der Einstich ist also weniger invasiv. Außerdem ist es durch die Einstellbarkeit des Abstands f möglich, bei verschiedenen Anwendungsfällen nur eine Art von Lichtapplikator einzusetzen, d.h. der Abstand d ist für alle Lichtapplikatoren unabhängig von der Anwendung identisch. Es müssen also nicht unterschiedliche Längen von Lichtapplikatoren für unterschiedliche Anwendungsfälle bereitgehalten und entsprechend dem Anwendungsfall korrekt ausgewählt werden. Nachdem die Platzierungsschablone derart in einer definierten Position zum organischen Körper platziert und fixiert ist, ist ein Sollpunkt für die Platzierung der Applikatorspitze im Körper fest definiert. Das Lagerpunktelement kann dann so weit wie möglich an den Körper verschoben werden, beispielsweise bis zum Hautkontakt, um einen möglichst großen Abstand f zu erzielen. Durch den damit platzierten Lagerpunkt wird das Risiko des Durchbiegens der Lichtapplikatoren stark verringert. Es ist auch denkbar, zuerst das Lagerpunktelement sogleich an seiner finalen Position zu platzieren - vorzugsweise so nahe wie möglich am Körper bzw. an der Haut - und an dieser Stelle zu fixieren und erst anschließend die Platzierungsschablone in den entsprechenden Abstand f vom Lagerpunktelement und damit in die definierte, finale Position zum organischen Körper zu bringen.

Optional kann der Lagerpunkt durch eine Öffnung im Lagerpunktelement gebildet sein, durch welche der jeweilige Lichtapplikator passgenau durchsteckbar ist.

Optional kann das Lagerpunktelement zumindest entlang der Einstichachse relativ zur Platzierungsschablone beweglich gelagert und positioniert arretierbar sein bzw. - bei einer gegenüber der finalen Platzierung der Platzierungsschablone vorhergehenden finalen Platzierung und Fixierung des Lagerpunktelements nahe am Körper- umgekehrt die Platzierungsschablone entlang der Einstichachse relativ zum Lagerpunktelement beweglich gelagert und positioniert arretierbar sein.

Optional kann das Lagerpunktelement derart geführt gelagert mit der Platzierungsschablone gekoppelt sein, dass das Lagerpunktelement relativ zur Platzierungsschablone ausschließlich linear entlang der Einstichachse beweglich ist bzw. - bei einer gegenüber der finalen Platzierung der Platzierungsschablone vorhergehenden finalen Platzierung und Fixierung des Lagerpunktelements nahe am Körper- umgekehrt die Platzierungsschablone derart geführt gelagert mit dem Lagerpunktelement gekoppelt sein, dass die Platzierungsschablone ausschließlich linear entlang der Einstichachse beweglich ist.

Optional kann das Lagerpunktelement als Lochplatte mit Löchern ausgeführt sein, wobei jedes Loch einen Lagerpunkt definiert, wobei die Löcher koaxial zu Lichtapplikatoraufnahmen der Platzierungsschablone angeordnet oder anordbar sind.

Optional kann das Lagerpunktelement eine von der Platzierungsschablone abgewandte Hautauflagefläche aufweisen, wobei die Hautauflagefläche auf Haut eines organischen Körpers auflegbar und dort positioniert fixierbar ist. Die Hautauflagefläche kann beispielsweise zumindest teilweise eine Klebe- oder Haftfläche sein, mit der das Lagerpunktelement für die Zeit der Behandlung auf die Haut aufgeklebt werden kann oder an dieser anhaftet. Vorzugsweise ist die Hautauflagefläche nur in rings um den Lagerpunkten herum angeordneten separaten Bereichen klebend oder haftend, um Klebefläche zu minimieren und das spätere Abziehen von der Haut zu vereinfachen.

Optional kann das Lagerpunktelement auch in eine Ebene quer zu Einstichachse relativ zur Platzierungsschablone positionierbar beweglich sein bzw. - bei einer gegenüber der finalen Platzierung der Platzierungsschablone vorhergehenden finalen Platzierung und Fixierung des Lagerpunktelements nahe am Körper- umgekehrt die Platzierungsschablone auch in einer Ebene quer zur Einstichachse relativ zum Lagerpunktelement positionierbar beweglich sein.

Optional können die Lichtapplikatoren unabhängig voneinander einzeln in die Platzierungsschablone und das Lagerpunktelement einsetzbar sein.

Optional kann die Platzierungsschablone mindestens einen ersten Schablonenteil aufweisen, der eine erste Untergruppe der Fixierpunktaufnahmen definiert, und mindestens einen zweiten Schablonenteil aufweisen, der eine zweite Untergruppe der Fixierpunktaufnahmen definiert, wobei der erste Schablonenteil relativ zum zweiten Schablonenteil entlang der Einstichachse geführt verschiebbar ist.

Optional kann die erste Untergruppe der Fixierpunktaufnahmen ein erstes Fixierpunktaufnahmeteilgitter und die zweite Untergruppe der Fixierpunktaufnahmen ein zweites Fixierpunktaufnahmeteilgitter definieren, wobei das zweite Fixierpunktaufnahmeteilgitter quer zur Einstichachse um einen lateralen Gitterversatz versetzt zum ersten Fixierpunktaufnahmeteilgitter ist.

Optional kann die erste Untergruppe der Fixierpunktaufnahmen, die zweite Untergruppe der Fixierpunktaufnahmen sowie die Lichtapplikatoren zusammen eine dreidimensionale Fixierpunktgitterstruktur definieren, wobei die Fixierpunktgitterstruktur einer virtuellen organspezifischen Sollpunktgitterstruktur für die lichtemittierenden Applikatorspitzen im Organ oder Organsegment entspricht und um einen Abstand d entlang der Einstichachse parallelverschoben zur Sollpunktgitterstruktur ist, wobei jeder Lichtapplikator mindestens einen definierten Fixierpunkt im Abstand d von der lichtemittierenden Applikatorspitze aufweist.

Optional kann der zweite Schablonenteil in proximale Richtung relativ zum ersten Schablonenteil nur bis zur Anlage an den ersten Schablonenteil verschiebbar sein, wobei bei Anlage des zweiten Schablonenteils am ersten Schablonenteil die vom ersten Schablonenteil definierte erste Untergruppe der Fixierpunktaufnahmen distal von der vom zweiten Schablonenteil definierten zweiten Untergruppe der Fixierpunktaufnahmen liegt.

Optional kann der erste Schablonenteil eine erste Untergruppe der Fixierpunktaufnahmen einer ersten Fixierpunktaufnahmegitterfläche und der zweite Schablonenteil eine zweite Untergruppe der Fixierpunktaufnahmen einer zweiten Fixierpunktaufnahmegitterfläche aufweisen.

Optional können die Lichtapplikatoren jeweils mindestens einen Fixierpunkt aufweisen, der durch einen Anschlag ausgebildet und arretierbar ist.

Vorzugsweise wird mit dem vorausgehend beschriebenen Aufbau angestrebt, mit der Realisierung und Positionierung der Platzierungsschablone außerhalb des Körpers in einem ersten Schritt eine virtuelle dreidimensionale Sollpunktgitterstruktur mittels einer Parallelverschiebung bzw. Translation um die Strecke d auf eine dreidimensionale Fixierpunktgitterstruktur abzubilden. Die virtuelle dreidimensionale Sollpunktgitterstruktur im Körper des Patienten repräsentiert einerseits die definierte Soll-Anordnung der lichtemittierenden Applikatorspitzen im Organ oder Organsegment im Hinblick auf eine lückenlose Bestrahlung des Organs oder Organsegments. Die Bedienperson soll also die Sollpunkte der Sollpunktgitterstruktur mit den lichtemittierenden Applikatorspitzen besetzen. Allerdings sind die Sollpunkte der Sollpunktgitterstruktur im Patientenkörper für die Bedienperson nicht sichtbar und deshalb zunächst nicht gezielt und direkt mit den lichtemittierenden Applikatorspitzen ansteuerbar. Die von der Platzierungsschablone gebildete Fixierpunktgitterstruktur, welche von den Fixierpunktaufnahmen in bzw. an der Platzierungsschablone außerhalb des Patientenkörpers repräsentiert wird, ist im Gegensatz zu den innerhalb des Körpers liegenden virtuellen Sollpunkten der virtuellen Sollpunktgitterstruktur für die Bedienperson sichtbar und mit dem mindestens einen Fixierpunkt eines jeweiligen Lichtapplikators gezielt und direkt ansteuerbar. Nach dem ersten Schritt der Parallelverschiebung bzw. Translation werden also in einem zweiten Schritt die lichtemittierenden Applikatorspitzen mittels einer in Bezug auf die oben beschriebene Parallelverschiebung inversen Parallelverschiebung an den virtuellen und für die Bedienperson unsichtbaren Sollpunkten der virtuellen Sollpunktgitterstruktur im Organ oder Organsegment in definierter, eindeutiger und damit sicherer Weise platziert. Das im einstellbaren Abstand f distalwärtig von der Platzierungsschablone platzierbare Lagerpunktelement reduziert dann insbesondere im Falle von größeren Abständen zwischen Platzierungsschablone und Haut des Patienten das Risiko, dass sich die dünnen Lichtapplikatoren beim Einstich in die Haut verbiegen.

In einem ersten Schritt kann also die virtuelle, nicht sichtbare und deshalb nicht direkt ansteuerbare, organspezifische Sollpunktgitterstruktur innerhalb des Körpers mittels einer Parallelverschiebung bzw. Translation in bzw. auf die Fixierpunktgitterstruktur, repräsentiert durch die real existierenden, sichtbaren und deshalb direkt zugänglichen Fixierpunktaufnahmen der Platzierungsschablone, außerhalb des Körpers transformiert bzw. abgebildet werden.

Nach diesem ersten Schritt folgt als zweiter Schritt die Positionierung des Lagerpunktelements im möglichst großen Abstand f distalseitig von der Platzierungsschablone bzw. in möglichst kleinem Abstand zur Haut des Patienten. Es ist dabei auch die umgekehrte Vorgehensweise denkbar, also die gegenüber der Platzierung der Platzierungsschablone vorhergehende Platzierung und Fixierung des Lagerpunktelements in möglichst kleinem Abstand zur Haut und die anschließende Platzierung und Fixierung der Platzierungsschablone in einem entsprechenden Abstand f zum Lagerpunktelement. Sodann folgt die Durchführung der eigentlichen Therapievorbereitung durch die Bedienperson, welche in der ordnungsgemäßen Verteilung und Platzierung der einzelnen Lichtapplikatoren bzw. der lichtemittierenden Applikatorspitzen im Patienten im Hinblick auf eine lückenlose Bestrahlung des Organs oder Organsegments besteht. Bei diesem zweiten Schritt werden die einzelnen lichtemittierenden Applikatorspitzen - ausgehend von den sichtbaren und direkt ansteuerbaren Fixierpunktaufnahmen der Platzierungsschablone außerhalb des Körpers - mittels der Parallelverschiebung, welche zur Parallelverschiebung des ersten Schrittes invers ist, in definierter, eindeutiger und damit sicherer Weise zu den unsichtbaren Sollpunkten innerhalb des Körpers transformiert bzw. bewegt. Die Definiertheit und Eindeutigkeit bei der Platzierung der einzelnen Applikatorspitzen in den Sollpunkten wird dadurch erreicht, dass erstens die Platzierungsschablone mit parallel zueinander ausgerichteten Führungen für eine definierte, eindeutige, parallele Ausrichtung der einzelnen Lichtapplikatoren versehen ist und dass zweitens einerseits die Platzierungsschablone mit Fixierpunktaufnahmen und andererseits die Lichtapplikatoren mit mindestens einem Fixierpunkt versehen sind, mit welchem die einzelnen Lichtapplikatoren in bzw. an den Fixierpunktaufnahmen der Platzierungsschablone an einer definierten und eindeutigen Stelle fixiert werden können, wodurch eine definierte und eindeutige Eindringtiefe der einzelnen Lichtapplikatoren in das Organ bzw. Organsegment erreicht wird. Dabei werden die Lichtapplikatoren in den jeweiligen Lagerpunkten des Lagerpunktelements zusätzlich zu den Führungen der Platzierungsschablone gelagert. Durch den eingestellten Abstand f wird das Risiko einer Durchbiegung der einzelnen Lichtapplikatoren stark reduziert.

Die Lichtapplikatoren können dabei besonders dünn und starr als Metallschaft und/oder -rohr ausgestaltet werden, da nur die Applikatorspitze lichtemittierend ausgestaltet werden muss. An der Applikatorspitze kann beispielsweise eine lichtemittierende Komponente in Form einer Lichtleiterauskopplung oder einer LED angeordnet sein. Die Applikatorspitze kann als lichttransparenter Streukörper distalseitig von der lichtemittierenden Komponente angeordnet sein. Das System zur Durchführung einer transkutanen PDT ist damit wesentlich gewebeschonender und weniger invasiv als aus dem Stand der Technik bekannte Systeme. Durch die dreidimensionale Fixierpunktgitterstruktur, welche durch die oben erläuterte Parallelverschiebung um die Länge d entlang der Einstichachse aus der Sollpunktgitterstruktur hervorgeht, mit einerseits Fixierpunktaufnahmen, die entsprechend dieser dreidimensionalen Fixierpunktgitterstruktur angeordnet sind, und mit andererseits Führungen, die beide zusammen (Fixierpunktaufnahme und Führung) in der Kombination mit dem mindestens einen Lichtapplikator-Fixierpunkt eine Bewegung und finale Platzierung der einzelnen Lichtapplikatoren und der lichtemittierenden Applikatorspitzen im Körper und im Organ ausschließlich gemäß einer der oben erläuterten Parallelverschiebung inversen Parallelverschiebung zulassen, und in diesem Sinne die diesbezüglichen Freiheitsgrade der Bedienperson auf ein Minimum reduzieren, wird - in Kombination mit der körpernahen Platzierung des erfindungsgemäß verstellbaren und auf diese Weise führungsverstärkenden Lagerpunktelements - sichergestellt, dass die einzelnen lichtemittierenden Applikatorspitzen entsprechend der Sollpunktgitterstruktur im Organ oder Organsegment so angeordnet sind, dass das Organ oder Organsegment lückenlos über das gesamte Volumen der Sollpunktgitterstruktur bestrahlt wird. Jeder Gitterpunkt der Sollpunktgitterstruktur wird dabei vorzugsweise von einer Applikatorspitze besetzt. Dies muss allerdings nicht unbedingt zeitgleich erfolgen. Eine besonders schnelle Durchführung der transkutanen PDT wird dadurch erreicht, dass sämtliche Gitterpunkte der Sollpunktgitterstruktur jeweils mit einer Applikatorspitze besetzt werden und dann zeitgleich das gesamte Organ oder Organsegment bestrahlt wird. Alternativ dazu kann die transkutane PDT sukzessive für unterschiedliche Gitterpunkte oder Gitterpunktgruppen der Sollpunktgitterstruktur durchgeführt werden. Dadurch kann, unter Inkaufnahme einer längeren Behandlungszeit, die Anzahl der benötigten Lichtapplikatoren und/oder Einstiche reduziert werden, wodurch die Patientenbelastung und die Kosten für die Behandlung ggf. reduziert werden können.

Beispielsweise kann die Sollpunktgitterstruktur mehrere Sollpunktgitterflächen aufweisen, die entlang der Einstichachse einen Gitterflächenabstand k aufweisen. Die Sollpunktgitterflächen können sich flach als Sollpunktgitterebenen oder gekrümmt als Teil der Sollpunktgitterstrukturerstrecken. Die perkutane PDT kann dann sukzessive füreine Sollpunktgitterfläche nach der anderen durchgeführt werden. Beispielsweise kann eine Bedienperson mit einer am tiefsten gelegenen Sollpunktgitterfläche beginnen und sich dann durch entsprechendes proximalwärtiges Herausziehen der einzelnen Lichtapplikatoren um jeweils einen Gitterflächenabstand k Sollpunktgitterfläche für Sollpunktgitterfläche vorarbeiten, bis die gesamte Sollpunktgitterstruktur bestrahlt wurde und damit das Organ oder Organsegment lückenlos bestrahlt wurde.

Die virtuelle organspezifische Sollpunktgitterstruktur liegt im Inneren des organischen Körpers überdas Organ oder Organsegment verteilt und ist damit für die Bedienperson nicht sichtbar. Die dreidimensionale Fixierpunktgitterstruktur hingegen, die vorzugsweise durch die Platzierungsschablone mit ihren Fixierpunktaufnahmen und die einzelnen Lichtapplikatoren mit ihren Fixierpunkten in Kombination miteinander definiert wird, liegt außerhalb des Körpers für die Bedienperson einsehbar und stellt - in Kombination mit der körpernahen Platzierung des erfindungsgemäß verstellbaren und auf diese Weise führungsverstärkenden Lagerpunktelements - sicher, dass die einzelnen lichtemittierenden Applikatorspitzen sich dann an den entsprechenden Gitterpunkten der Sollpunktgitterstruktur befinden, wenn sich der Fixierpunkt des jeweiligen Lichtapplikators am zugehörigen Gitterpunkt der Fixierpunktgitterstruktur, d.h. in der entsprechenden Fixierpunktaufnahme der Platzierungsschablone, befindet.

Eine Bedienperson kann beispielsweise durch eine Führung, die sich erstens zentral in der Platzierungsschablone befindet und deren zugehörige Fixierpunktaufnahme zweitens weitgehend distal in der Platzierungsschablone angeordnet ist, einen ersten Lichtapplikator in das Organ oder Organsegment einstechen. Vorzugsweise befinden sich dabei die Platzierungsschablone, das der Platzierungsschablone distal vorgelagerte Lagerpunktelement und die Haut des Patienten zunächst in minimalem Abstand zueinander. Außerdem wird oder ist das Lagerpunktelement gegenüber der Platzierungsschablone derart platziert, dass seine Lagerpunkte koaxial zu den entsprechenden Lichtapplikatoraufnahmen an der Platzierungsschablone angeordnet sind. Die Bedienperson kann dann mittels eines bildgebenden Verfahrens, beispielsweise mittels Ultraschall-Sonographie, die Applikatorspitze dieses ersten Lichtapplikators an einem dieser Fixierpunktaufnahme entsprechenden Gitterpunkt der Sollpunktgitterstruktur im Organ oder Organsegment, d.h. gleichfalls weitgehend distal und innerhalb dieser ausgewählten, distalen Sollpunktgitterebene gleichfalls zentral angeordnet, platzieren. Der zunächst jeweils minimale Abstand von Platzierungsschablone, Lagerpunktelement und Haut zueinander und die so erreichte bestmögliche Führung des Lichtapplikators beim Durchdringen der Haut sorgen dafür, dass sich der erste Lichtapplikator während des Einführens in den Körper bzw. durch die Haut des Patienten nicht verbiegen kann. Der Körper des Patienten ist dabei vorzugsweise relativ zu einer festen Referenzfläche, beispielsweise einem Behandlungstisch, fixiert. Die Platzierungsschablone kann dann bei korrekt positioniertem ersten Lichtapplikator bzw. dessen lichtemittierender Applikatorspitze und unter Aufrechterhaltung dieser ausgewählten Lichtapplikator-Position relativ zu diesem entlang der Einstichachse verschoben und so positioniert werden, dass sich der Fixierpunkt des ersten Lichtapplikators am entsprechenden Gitterpunkt der Fixierpunktgitterstruktur befindet. Bei der oben beschriebenen Vorgehensweise mit zunächst minimalem Abstand der drei Komponenten Platzierungsschablone, Lagerpunktelement und Haut zueinander geschieht dies dadurch, dass die Platzierungsschablone weg von der Haut in proximale Richtung zurückgezogen wird. In dieser Position kann sowohl der Fixierpunkt des Lichtapplikators an der Fixierpunktaufnahme der Platzierungsschablone als auch die Platzierungsschablone an der äußeren Referenzfläche, beispielsweise einem Behandlungstisch, fixiert werden.

Mit diesem Vorgang wird die dreidimensionale Fixierpunktgitterstruktur in Bezug auf den Körper des Patienten räumlich festgelegt. Zudem wird mit diesem Vorgang, durch welchen also der definierte Abstand d zwischen virtueller Sollpunktgitterstruktur und Fixierpunktgitterstruktur hergestellt wird, auch der erste Schritt bei dieser Vorgehensweise, nämlich die erste Abbildung, d.h. die Parallelverschiebung bzw. Translation der virtuellen Sollpunktgitterstruktur im Organ bzw. Organsegment innerhalb des menschlichen Körpers in einen Bereich außerhalb des menschlichen Körpers abgeschlossen.

Es wird nun vorzugsweise dafür gesorgt, dass das Lagerpunktelement - im Gegensatz zur neu positionierten Platzierungsschablone in entsprechendem Abstand zur Haut des Patienten - auch dann in minimalem Abstand zur Haut des Patienten bleibt, wenn die restlichen Lichtapplikatoren einzeln in den Körper eingeführt werden, um so auch für diese restlichen Applikatoren eine optimale Führung (kein Verbiegen) beim Durchdringen der Haut zu gewährleisten. Entweder wird der minimale Abstand des Lagerpunktelements zur Haut bereits während des Zurückziehens der Platzierungsschablone in proximale Richtung aufrechterhalten. Im anderen Fall, dass das Lagerpunktelement konstruktiv bedingt zunächst zusammen mit der Platzierungsschablone in proximale Richtung zurückgezogen wird, wird das Lagerpunktelement anschließend wieder an seine ursprüngliche Position nahe der Haut des Patienten in distale Richtung zurückbewegt.

Die Platzierungen der einzelnen weiteren Lichtapplikatoren ist nun besonders einfach, fehlerfrei und schnell durchführbar, da sie nur durch die entsprechenden Führungen der Platzierungsschablone und den jeweiligen Lagerpunkt des Lagerpunktelements ohne Verbiegung eingestochen werden müssen und soweit vorgeschoben werden müssen, bis sich ihr Fixierpunkt im entsprechenden Gitterpunkt der Fixierpunktgitterstruktur, d.h. in der Fixierpunktaufnahme der Platzierungsschablone befindet. Automatisch befindet sich dann die Applikatorspitze am zugehörigen Gitterpunkt der Sollpunktgitterstruktur. Die Platzierung des Lagerpunktelements in maximalem Abstand zur Platzierungsschablone bzw. in minimalem Abstand zur Haut des Patienten gewährleistet dabei, dass die einzelnen Applikatoren beim Einführen durch die Haut nicht verbogen werden und dadurch die zugehörigen Gitterpunkte der Sollpunktgitterstruktur zielgenau getroffen werden, auch dann, wenn sich die Platzierungsschablone in einem größeren Abstand zur Haut befindet. Die virtuelle organspezifische Sollpunktgitterstruktur stellt sicher, dass zum einen die einzelnen Applikatorspitzen nicht zu weit voneinander entfernt sind, sodass keine Bestrahlungslücken im Organ oder Organsegment entstehen, und zum anderen nicht zu nah zueinander angeordnet sind, um den Patienten nicht mit unnötig vielen Einstichen belasten zu müssen. Die Sollpunktgitterstruktur ist organspezifisch, da die äußere Form und Größe der Sollpunktgitterstruktur vom Organ oder Organsegment abhängt. Zudem hängt die Dichte der Sollpunkte der Sollpunktgitterstruktur davon ab, wie groß die Eindringtiefe des Lichts in das Gewebe des jeweiligen Organs oder Organsegments ist. Je geringer die Eindringtiefe des Bestrahlungslichts ist, umso dichter muss die Sollpunktgitterstruktur gewählt werden, um eine lückenlose Bestrahlung des Organs oder Organsegments sicherzustellen.

Optional kann die Versorgungseinheit dazu eingerichtet sein, die Lichtapplikatoren mit Strom zu versorgen, wobei jeder Lichtapplikator am distalen Ende des Einführabschnitts eine mit dem Strom betreibbare LED und/oder eine andere lichtemittierende Komponente aufweist. Gegenüber einer Lichtleiterauskopplung hat eine LED und/oder eine andere entsprechende lichtemittierende Komponente am distalen Ende des Einführabschnitts den großen Vorteil, dass kein teurer Laser benötigt wird, dessen Licht über die Versorgungseinheit in den Lichtleiter eingekoppelt werden muss. Da die Versorgungseinheit die einzelnen Lichtapplikatoren lediglich mit Strom versorgen muss, kann sie besonders einfach und günstig ausgestaltet sein. Die LED und/oder die andere lichtemittierende Komponente weist vorzugsweise ein auf den Photosensibilisator bzw. Markerstoff abgestimmtes Leuchtspektrum auf. Alternativ oder zusätzlich kann dazu ein Lichtfilter eingesetzt werden.

Optional sind die Fixierpunktaufnahmen jeweils in oder an einer zugehörigen Führung angeordnet oder werden von dieser gebildet.

Optional können die einzelnen Lichtapplikatoren jeweils mindestens einen Fixierpunkt aufweisen, der durch einen Anschlag ausgebildet ist und entsprechend der dreidimensionalen Fixierpunktgitterstruktur an einer Fixierpunktaufnahme der Platzierungsschablone arretierbar ist. Durch den Anschlag und die Arretierung erhält eine Bedienperson ein haptisches, akustisches und/oder optisches Feedback über die korrekte Platzierung des Fixierpunkts an einem Gitterpunkt der Fixierpunktgitterstruktur bzw. der entsprechenden Fixierpunktaufnahme.

Optional kann die virtuelle organspezifische Sollpunktgitterstruktur eine Mehrzahl von räumlich auf Sollpunktgitterflächen verteilt angeordneten Sollpunkten aufweisen, wobei die Sollpunktgitterflächen entlang der Einstichachse einen Abstand k zueinander haben. Vorzugsweise erstrecken sich die Sollpunktgitterflächen quer zur Einstichachse.

Optional können die einzelnen Lichtapplikatoren jeweils mindestens zwei Fixierpunkte aufweisen, welche zueinander entlang der Einstichachse den Abstand k haben, den auch die Sollpunktgitterflächen entlang der Einstichachse zueinander haben. Dadurch hat die Bedienperson die Möglichkeit, einen der Fixierpunkte auszuwählen und am entsprechenden Gitterpunkt der Fixierpunktgitterstruktur bzw. der zugehörigen Fixierpunktaufnahme der Platzierungsschablone zu fixieren und dadurch die Sollpunktgitterfläche festzulegen, in der die Applikatorspitze liegen soll.

Optional kann die Platzierungsschablone eine Mehrzahl von räumlich auf Fixierpunktaufnahmegitterflächen verteilt angeordneten Fixierpunktaufnahmen aufweisen, wobei die Fixierpunktaufnahmegitterflächen entlang der Einstichachse den Abstand k zueinander haben, den auch die entsprechenden Sollpunktgitterflächen entlang der Einstichachse zueinander haben. Eine Bedienperson hat dabei die Möglichkeit, durch Auswahl der Fixierpunktaufnahme, in der der Fixierpunkt arretiert werden soll, die Sollpunktgitterfläche festzulegen, in welcher die Applikatorspitze liegen soll.

Optional kann die virtuelle organspezifische Sollpunktgitterstruktur eine Mehrzahl von räumlich auf Sollpunktgitterflächen verteilt angeordneten Sollpunkten aufweisen, wobei mindestens acht der Sollpunkte Eckpunkte einer Gitterelementarzelle der Sollpunktgitterstruktur in Form eines Parallelepipeds bilden. Vorzugsweise weist dabei die Gitterelementarzelle drei Gitterelementarzellenkanten und vier Gitterelementarzellendiagonalen auf, von denen eine oder keine entlang der Einstichachse verläuft. Die Variante, bei der keine der drei Gitterelementarzellenkanten oder vier Gitterelementarzellendiagonalen entlang der Einstichachse verläuft, hat den Vorteil, dass mehrere Sollpunktgitterflächen gleichzeitig mit Applikatorspitzen besetzt werden können und somit die PDT schneller durchgeführt werden kann. Die Variante, bei der eine der drei Gitterelementarzellenkanten oder der vier Gitterelementarzellendiagonalen entlang der Einstichachse verläuft, hat dagegen den Vorteil, dass ein Einstich für mehrere Sollpunktgitterflächen verwendet werden kann und somit zum einen weniger Einstiche benötigt werden, was für den Patienten gewebeschonender ist, und zum anderen weniger Lichtapplikatoren benötigt werden, wodurch das System kostengünstiger wird.

Gemäß einem zweiten Aspekt der vorliegenden Offenbarung, der vorzugsweise mit dem ersten Aspekt kombinierbar oder unabhängig davon ist, wird ein System zur Durchführung einer transkutanen PDT in einem Organ oder Organsegment eines organischen Körpers bereitgestellt, wobei das System eine Mehrzahl von einzelnen Lichtapplikatoren, eine Versorgungseinheit zum Versorgen der Lichtapplikatoren mit Licht und/oder Strom und eine in einer definierten Position zum organischen Körper platzierbare Platzierungsschablone zum definierten Ausrichten, Platzieren und Fixieren der einzelnen Lichtapplikatoren aufweist. Die einzelnen Lichtapplikatoren weisen dabei jeweils einen nadelartigen Einführabschnitt zum transkutanen Einstechen entlang einer Einstichachse in das Organ oder Organsegment und am distalen Ende des Einführabschnitts eine lichtemittierende Applikatorspitze auf, sowie mindestens einen definierten Fixierpunkt in einem proximalen Abstand d von der Applikatorspitze. Zudem definiert die Platzierungsschablone eine Mehrzahl an Fixierpunktaufnahmen, mittels welcher die einzelnen Lichtapplikatoren mit ihrem mindestens einen definierten Fixierpunkt fixierbar sind. Die Platzierungsschablone kann dabei eine Mehrzahl an Führungen definieren, wobei durch jede der Führungen ein Einführabschnitt eines der Lichtapplikatoren geführt transkutan in das Organ oder Organsegment entlang der Einstichachse einstechbar ist. Die Fixierpunktaufnahmen können an oder in den Führungen ausgebildet sein.

Die Platzierungsschablone weist gemäß dem zweiten Aspekt mindestens einen ersten Schablonenteil auf, der eine erste Untergruppe der Fixierpunktaufnahmen definiert, und mindestens einen zweiten Schablonenteil, der eine zweite Untergruppe der Fixierpunktaufnahmen definiert, wobei der erste Schablonenteil relativ zum zweiten Schablonenteil entlang der Einstichachse geführt verschiebbar ist.

Durch die Schablonenteile können verschiedene Untergruppen von Lichtapplikatoren, mittels Fixierung in entsprechenden Untergruppen von Fixierpunktaufnahmen, gemeinsam konzertiert wahlweise bewegt oder festgehalten werden. Dadurch wird es möglich, mit einer Untergruppe von Lichtapplikatoren das Organ oder Organsegment festzuhalten bzw. seine Form aufrecht zu erhalten, während die andere Untergruppe bewegt wird.

Ein solches System und eine solche Vorgehensweise können aus nachfolgend genanntem Grund vorteilhaft sein. Werden ein in das Organ oder Organsegment eingeführter Lichtapplikator bewegt bzw. verschoben, dann übt dieser auf das Organ oder Organsegment bzw. auf das Organgewebe, welches am Lichtapplikator anliegt, eine Haftreibungskraft aus. Werden die Lichtapplikatoren alle gemeinsam bzw. gleichzeitig in eine Richtung bewegt, dann ist auch die Summe dieser Haftreibungskräfte auf das Organ oder Organsegment in diese Richtung entsprechend groß. Abhängig von den unterschiedlichen, nachfolgend erläuterten Randbedingungen können dann zwei prinzipiell unterschiedliche, relevante Fälle eintreten, die auch in kombinierter Form auftreten können.

In einem ersten Fall, welcher auf der Randbedingung basiert, dass die beim gemeinsamen bzw. gleichzeitigen Bewegen der Lichtapplikatoren in eine Richtung auf das Organ bzw. Organsegment wirksame Gesamthaftreibungskraft größer ist als die Bindungskräfte, die innerhalb des umgebenden, mit dem zu therapierenden Organ oder Organsegment direkt verbundenen Gewebe wirken und wiederum kleiner sind als die innerhalb des Organs oder Organsegments selbst wirksamen Bindungskräfte, kann das Organ oder Organsegment von den gemeinsam bzw. gleichzeitig in eine gemeinsame Richtung bewegten Lichtapplikatoren als Ganzes und in seiner Form unverändert "mitgenommen" werden, d.h. das Organ oder Organsegment hält beim gemeinsamen bzw. gleichzeitigen Verschieben der Lichtapplikatoren in eine Richtung unter den vorausgehend genannten Randbedingungen zwar seine Form aufrecht, verändert aber seine Position im Körper. In diesem ersten Fall würde dann zwar die Sollpunktgitterstruktur in ihrer Form unverändert bleiben und dadurch weiterhin der Form der Fixierpunktgitterstruktur entsprechen, allerdings würde sich die Lage der Sollpunktgitterstruktur gegenüber der weiterhin unveränderten Lage der Fixierpunktgitterstruktur ändern, d.h. die Fixierpunktgitterstruktur wäre in unerwünschter Weise nicht mehr länger um die ursprüngliche, die Parallelverschiebung kennzeichnende Länge d entlang der Einstichachse, welche dem Abstand d zwischen Applikatorspitze und Fixierpunkt entspricht, parallelverschoben.

In einem zweiten Fall, welcher auf der Randbedingung basiert, dass die beim gemeinsamen bzw. gleichzeitigen Bewegen der Lichtapplikatoren in eine Richtung auf das Organ oder Organsegment wirksame Gesamthaftreibungskraft größer ist als die innerhalb des Organs oder Organsegments selbst wirksamen Bindungskräfte, welche wiederum kleiner sind als die Bindungskräfte, die innerhalb des umgebenden, mit dem zu therapierenden Organ oder Organsegment direkt verbundenen Gewebe wirken, kann das Organ oder Organsegment in Bewegungsrichtung der Lichtapplikatoren gedehnt bzw. gestreckt werden, d.h. das Organ oder Organsegment hält beim gemeinsamen bzw. gleichzeitigen Verschieben der Lichtapplikatoren in eine Richtung unter den vorausgehend genannten Randbedingungen zwar seine Position im Körper in erster Näherung aufrecht, verändert aber seine Form. In diesem zweiten Fall wäre dann zwar die Lage der Sollpunktgitterstruktur gegenüber der Lage der Fixierpunktgitterstruktur in erster Näherung unverändert, d.h. der Abstand zwischen den beiden Gitterstrukturen entspräche nach dem gemeinsamen bzw. gleichzeitigen Verschieben der Lichtapplikatoren in erster Näherung noch der die Parallelverschiebung kennzeichnenden Länge d, allerdings würde sich mit der Form des Organs in gleicher Weise die Form der Sollpunktgitterstruktur ändern, die wiederum dann aber nicht mehr länger der Form der Fixierpunktgitterstruktur entspräche.

In beiden Fällen, oder einer Kombination daraus, wäre ein solcher "Aufgabeleffekt" negativ, da, mathematisch-geometrisch betrachtet, in beiden Fällen die ursprüngliche Parallelverschiebung um die Länge d, durch welche die Fixierpunktgitterstruktur aus der Sollpunktgitterstruktur hervorging und welche die Basis der hier dargestellten Vorgehensweise für eine vollständige und vor allen Dingen punktgenaue Behandlung des Organs oder Organsegments bildet, beeinträchtigt bzw. modifiziert würde, d.h. kennzeichnende Parameter der Parallelverschiebung (Objekt-Form, Verschiebungslänge d) verändert würden und deshalb eine gleichmäßige und vor allen Dingen lückenlose Bestrahlung nicht mehr automatisch gewährleistet wäre.

Beide erläuterten Fälle des "Aufgabeleffekts" bzw. deren Auswirkungen gewinnen mit zunehmender Anzahl von Lichtapplikatoren an Bedeutung, weil jeder zusätzliche Lichtapplikator an seiner Kontaktfläche mit dem Organ oder Organsegment, welche im Wesentlichen durch den Lichtapplikator-Schaft gebildet wird, eine zusätzliche Haftreibungskraft auf das Organ oder Organsegment ausübt, welche die Gesamthaftreibungskraft weiter erhöht. Damit steigt mit zunehmender Anzahl von Lichtapplikatoren die Wahrscheinlichkeit, dass die Gesamthaftreibungskraft entweder die Bindungskräfte innerhalb des umgebenden, mit dem Organ direkt verbundenen Gewebes übersteigt (erster oben beschriebener Fall) und/oder die Bindungskräfte innerhalb des Organs oder Organsegments selbst übersteigt (zweiter oben beschriebener Fall) und daraus entweder eine Verschiebung des Organs oder Organsegments in proximale Richtung (erster oben beschriebener Fall), und/oder eine Verformung des Organs oder Organsegments (zweiter oben beschriebener Fall) resultiert.

Der "Aufgabeleffekt" kann dadurch vermieden werden, dass eine Untergruppe von Lichtapplikatoren festgehalten wird während die andere bewegt bzw. verschoben bzw. herausgezogen wird. Die jeweiligen Untergruppen können abwechselnd bewegt werden, während die andere festgehalten wird. Die gemeinsame Bewegung und das gemeinsame Festhalten der Lichtapplikatoren kann durch entsprechende Bewegung bzw. Festhalten der Schablonenteile bewirkt werden.

Optional kann die erste Untergruppe der Fixierpunktaufnahmen ein erstes Fixierpunktaufnahmeteilgitter und die zweite Untergruppe der Fixierpunktaufnahmen ein zweites Fixierpunktaufnahmeteilgitter definieren, wobei das zweite Fixierpunktaufnahmeteilgitter quer zur Einstichachse um einen lateralen Gitterversatz versetzt zum ersten Fixierpunktaufnahmeteilgitter ist. Vorzugsweise hat dadurch jeder Lichtapplikator mindestens einen anderen Lichtapplikator als lateral nächstliegenden Nachbar, der in der jeweils anderen Untergruppe der Führungen steckt. Dadurch kann der "Aufgabeleffekt" besonders gut vermieden werden, weil sich die resultierenden Haftreibungskräfte benachbarter Lichtapplikatoren paarweise in erster Näherung aufheben und dadurch auch die Gesamthaftreibungskraft Null ergibt. Außerdem können dadurch auch lokale Verschiebungen und/ Verformungen des Organs oder Organsegments vermieden werden. Vorzugsweise weist das erste Fixierpunktaufnahmeteilgitter in etwa die gleiche Anzahl an Fixierpunktaufnahmen auf wie das zweite Fixierpunktaufnahmeteilgitter.

Optional kann der laterale Gitterversatz kleiner sein als eine Seitenlänge einer Gitterelementarzelle des ersten Fixierpunktaufnahmeteilgitters und/oder des zweiten Fixierpunktaufnahmeteilgitters. Vorzugsweise ist die Seitenlänge einer Gitterelementarzelle des ersten Fixierpunktaufnahmeteilgitters und des zweiten Fixierpunktaufnahmeteilgitters identisch und der laterale Gitterversatz beträgt die Hälfte der Seitenlänge einer Gitterelementarzelle. Der Gitterversatz kann vorzugsweise in zwei orthogonalen seitlichen Richtungen vorgesehen sein, sodass eine Fixierpunktaufnahme des ersten Fixierpunktaufnahmeteilgitters den Mittelpunkt einer Gitterzelle des zweiten Fixierpunktaufnahmeteilgitters bildet und umgekehrt.

Optional kann das erste Fixierpunktaufnahmeteilgitter und das zweite Fixierpunktaufnahmeteilgitter in einer Fixierpunktaufnahmegitterfläche liegen. Dadurch ergänzen sich die Fixierpunktaufnahmeteilgitter zu einem gesamten Fixierpunktaufnahmegitter in einer Fixierpunktaufnahmegitterfläche. Eine solche Anordnung hat - im Vergleich mit der nachfolgend beschriebenen Anordnung - den Vorteil, dass sich beim Bewegen bzw. Verschieben eines Schablonenteils, welcher eines der beiden Fixierpunktaufnahmeteilgitter repräsentiert, die in entgegengesetzte Richtungen zeigenden Reibungskräfte noch besser aufheben, weil die die Reibungskräfte bestimmenden Schaftlängen innerhalb des Organs oder Organsegments gleich oder ähnlich sind, weil sie zumindest zeitweise paarweise gleich weit in das Organ oder Organsegment hineinragen. Bei der angestrebten paarweisen Aufhebung der Reibungskräfte wird zunächst vereinfachend zu Grunde gelegt, dass beide Fixierpunktaufnahmegitterflächen mit der gleichen Anzahl von Applikatoren besetzt sind. Lägen die Fixierpunktaufnahmeteilgitter jedoch nicht in derselben Fixierpunktaufnahmegitterfläche, kann es sein, dass sich die Reibungskräfte nicht mehr vollständig paarweise kompensieren, da sich die jeweiligen Schaftlängen unterscheiden, welche sich innerhalb des Organs oder Organsegments befinden. Dies kann ganz oder teilweise dadurch korrigiert werden, dass das Fixierpunktaufnahmeteilgitter bzw. das entsprechende Schablonenteil, welches zuerst in proximale Richtung bewegt wird, mit einer höheren Anzahl von Lichtapplikatoren belegt wird.

Optional können die erste Untergruppe der Führungen und Fixierpunktaufnahmen, die zweite Untergruppe der Führungen und Fixierpunktaufnahmen sowie die Lichtapplikatoren zusammen eine dreidimensionale Fixierpunktgitterstruktur definieren, wobei die Fixierpunktgitterstruktur einer virtuellen organspezifischen Sollpunktgitterstruktur für die lichtemittierenden Applikatorspitzen im Organ oder Organsegment entspricht und um eine Länge d entlang der Einstichachse parallelverschoben zur Sollpunktgitterstruktur ist, wobei jeder Lichtapplikator mindestens einen definierten Fixierpunkt im Abstand d von der Applikatorspitze aufweist.

Optional kann die Versorgungseinheit dazu eingerichtet sein, die Lichtapplikatoren mit Strom zu versorgen und jeder Lichtapplikator kann am distalen Ende des Einführabschnitts eine mit dem Strom betreibbare LED oder eine andere lichtemittierende Komponente aufweisen. Gegenüber einer Lichtleiterauskopplung hat eine LED am distalen Ende des Einführabschnitts den großen Vorteil, dass kein teurer Laser benötigt wird, dessen Licht über die Versorgungseinheit in den Lichtleiter eingekoppelt werden muss. Da die Versorgungseinheit die Lichtapplikatoren lediglich mit Strom versorgen muss, kann sie besonders einfach und günstig ausgestaltet sein. Die LED weist vorzugsweise ein auf den Photosensibilisator bzw. Markerstoff abgestimmtes Leuchtspektrum auf. Alternativ oder zusätzlich kann dazu ein Lichtfilter eingesetzt werden.

Optional können die einzelnen Lichtapplikatoren jeweils mindestens einen Fixierpunkt aufweisen, der durch einen Anschlag ausgebildet und arretierbar ist. Durch den Anschlag und die Arretierung erhält eine Bedienperson ein haptisches, akustisches und/oder optisches Feedback über die korrekte Platzierung des Fixierpunkts an einem Gitterpunkt der Fixierpunktgitterstruktur bzw. der entsprechenden Fixierpunktaufnahme der Platzierungsschablone bzw. des Schablonenteils.

Optional kann die virtuelle organspezifische Sollpunktgitterstruktur eine Mehrzahl von räumlich auf Sollpunktgitterflächen verteilt angeordneten Sollpunkten aufweisen, wobei die Sollpunktgitterflächen entlang der Einstichachse einen Abstand k zueinander haben. Vorzugsweise erstrecken sich die Sollpunktgitterflächen orthogonal zur Einstichachse.

Optional können mindestens acht der Sollpunkte Eckpunkte einer Gitterelementarzelle der Sollpunktgitterstruktur in Form eines Parallelepipeds bilden. Optional kann dabei die Gitterelementarzelle drei Gitterelementarzellenkanten und vier Gitterelementarzellendiagonalen aufweisen, von denen eine entlang der Einstichachse verläuft. Dadurch ist es besonders einfach, alle im ersten Schablonenteil geführten Lichtapplikatoren durch Verschieben des ersten Schablonenteils zeitgleich gemeinsam so zu positionieren, dass die jeweiligen lichtemittierenden Applikatorspitzen von einer Sollpunktgitterfläche zur nächsten geführt werden. Währenddessen können die durch das zweite Schablonenteil geführten Lichtapplikatoren ortsfest gehalten werden und somit das Organ oder Organsegment festhalten und/oder seine Verformung unterdrücken, während die im ersten Schablonenteil geführten Lichtapplikatoren gemeinsam bewegt werden. Sobald die im ersten Schablonenteil geführten Lichtapplikatoren platziert sind, können diese ortsfest gehalten werden, während die im zweiten Schablonenteil geführten Lichtapplikatoren mit ihren lichtemittierenden Applikatorspitzen zur nächsten Sollpunktgitterfläche bewegt werden. Vorzugsweise beginnt dieser Prozess mit der distalwärtig am tiefsten im Körper gelegenen Sollpunktgitterfläche des jeweiligen Schablonenteils und man arbeitet sich dann durch Zurückziehen des jeweiligen Schablonenteils zur nächsten Sollpunktgitterfläche proximalwärts vor. Die PDT kann jeweils dann durchgeführt werden, wenn die Applikatorspitzen an ihrem Sollpunkt im Sollpunktgitter positioniert sind.

Optional kann eine mit der ersten Untergruppe der Führungen bzw. Fixierpunktaufnahmen korrespondierende erste Untergruppe der Sollpunkte ein erstes Sollpunktteilgitter und eine mit der zweiten Untergruppe der Führungen bzw. Fixierpunktaufnahmen korrespondierende zweite Untergruppe der Sollpunkte ein zweites Sollpunktteilgitter definieren, sodass sich das erste Sollpunktteilgitter und das zweite Sollpunktteilgitter zu einer der Sollpunktgitterflächen ergänzen. Auf diese Weise kann jede Sollpunktgitterfläche dadurch vollständig mit lichtemittierenden Applikatorspitzen besetzt werden, dass zunächst ein Sollpunktteilgitter mit lichtemittierenden Applikatorspitzen besetzt wird und sodann das andere Sollpunktteilgitter besetzt wird. Dies kann auf einfache Weise durch entsprechendes Verschieben der Schablonenteile relativ zueinander erfolgen.

Optional können die einzelnen Lichtapplikatoren jeweils mindestens zwei Fixierpunkte aufweisen, welche zueinander entlang der Einstichachse den Abstand k haben, den auch die Sollpunktgitterflächen zueinander haben. Damit kann die Bedienperson durch einen der Fixierpunkte, der im Gitterpunkt der Fixierpunktgitterstruktur bzw. in der entsprechenden Fixierpunktaufnahme des jeweiligen Schablonenteils arretiert werden soll, bestimmen, in welcher Sollpunktgitterfläche die lichtemittierende Applikatorspitze liegen soll.

Optional kann die Platzierungsschablone eine Mehrzahl von räumlich auf Fixierpunktaufnahmegitterflächen verteilt angeordneten Fixierpunktaufnahmen aufweisen, wobei die Fixierpunktaufnahmegitterflächen entlang der Einstichachse den Abstand k zueinander haben, den auch die Sollpunktgitterflächen zueinander haben. Damit kann eine Bedienperson durch Auswahl einer der Fixierpunktaufnahmen, in der ein Fixierpunkt arretiert werden soll, bestimmen, in welcher Sollpunktgitterfläche die Applikatorspitze liegen soll.

Optional kann der erste Schablonenteil eine erste Untergruppe der Fixierpunktaufnahmen einer ersten Fixierpunktaufnahmegitterfläche und der zweite Schablonenteil eine zweite Untergruppe der Fixierpunktaufnahmen einer zweiten Fixierpunktaufnahmegitterfläche aufweisen. Die beiden Untergruppen von Fixierpunktaufnahmen können sich daher über zwei Fixierpunktgitterflächen der Fixierpunktgitterstruktur ergänzen.

Optional kann der zweite Schablonenteil in proximale Richtung relativ zum ersten Schablonenteil nur bis zur Anlage an den ersten Schablonenteil verschiebbar sein, wobei bei Anlage des zweiten Schablonenteils am ersten Schablonenteil die vom ersten Schablonenteil definierte erste Untergruppe der Fixierpunktaufnahmen distal von der vom zweiten Schablonenteil definierten zweiten Untergruppe der Fixierpunktaufnahmen liegt.

Im Folgenden wird das hierin offenbarte System näheranhand der beiliegenden Figuren erläutert. Es zeigen:
- Fig. 1: eine schematische Darstellung der prinzipiellen Funktionsweise einer perkutanen PDT mit einem Lichtapplikator ohne Lagerpunktelement wie in Fig. 23 der zukünftig zu veröffentlichenden DE 10 2021 211 328 A1 und DE 10 2021 211 331 A1;
- Fig. 2: eine schematische Darstellung der prinzipiellen Funktionsweise einer perkutanen PDT ohne Lagerpunktelement wie in Fig. 30 der zukünftig zu veröffentlichenden DE 10 2021 211 328 A1 und DE 10 2021 211 331 A1;
- Fig. 3a,b: schematische Darstellungen der Problematik einer perkutanen PDT bei unterschiedlichen Positionen des zu behandelnden Organs oder Organsegments;
- Fig. 4a,b: schematische Darstellungen der Problematik einer perkutanen PDT bei relativ großen Volumen des zu behandelnden Organs oder Organsegments;
- Fig. 5a-h: schematische Darstellungen der Problematik einer perkutanen PDT beim Einstich in die Haut eines Patienten mit relativ dünnen Lichtapplikatoren;
- Fig.6: eine schematische Darstellung eines Ausführungsbeispiels eines hierin offenbarten Systems zur Durchführung einer transkutanen PDT mit schematisiert dargestelltem Lagerpunktelement;
- Fig.7: eine schematische Darstellung eines weiteren Ausführungsbeispiels eines hierin offenbarten Systems zur Durchführung einer transkutanen PDT mit Lagerpunktelement;
- Fig.8: eine schematische Darstellung eines weiteren Ausführungsbeispiels eines hierin offenbarten Systems zur Durchführung einer transkutanen PDT mit Lagerpunktelement;
- Fig.9a-c: schematische Darstellungen eines weiteren Ausführungsbeispiels eines hierin offenbarten Systems zur Durchführung einer transkutanen PDT mit Lagerpunktelement;
- Fig. 10a,b: schematische Darstellungen eines weiteren Ausführungsbeispiels eines hierin offenbarten Systems zur Durchführung einer transkutanen PDT mit Lagerpunktelement;
- Fig. 11: eine schematische Darstellung eines weiteren Ausführungsbeispiels eines hierin offenbarten Systems zur Durchführung einer transkutanen PDT mit Lagerpunktelement; und
- Fig. 12: eine schematische Querschnittsdarstellung des in Fig. 11 gezeigten Systems.

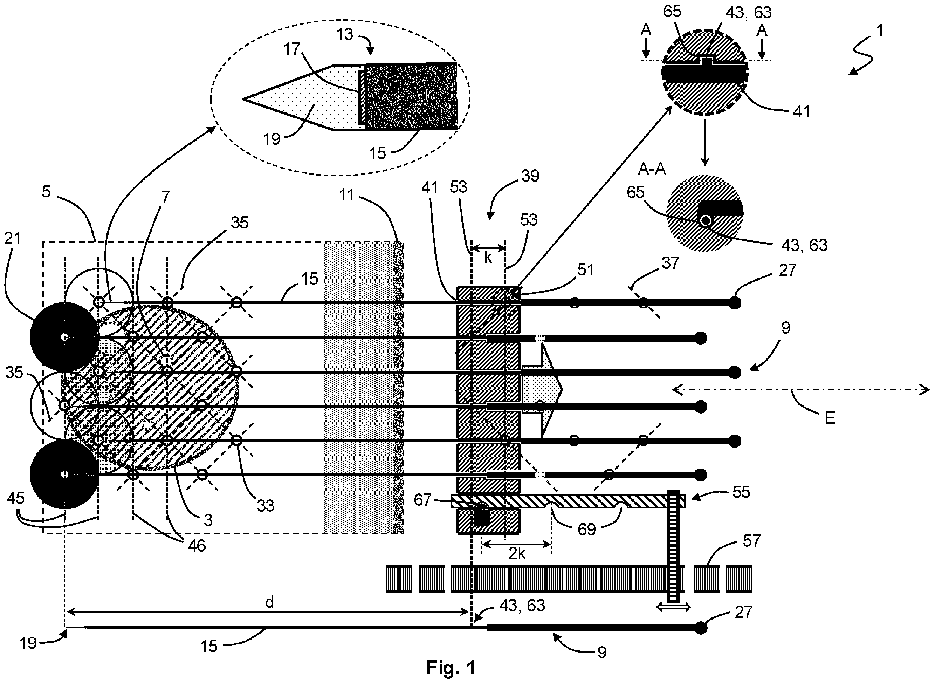

Fig. 1 zeigt ein System 1 zur Durchführung einer transkutanen photodynamischen Therapie (PDT) in einem Organ 3, beispielsweise der Prostata, eines organischen Körpers 5. Das Organ 3 weist hier mehrere pathologische Gewebebereiche 7 (in den Figuren durch eine gepunktete Umrandung gekennzeichnet) auf, in denen sich ein verabreichter Photosensibilisator bzw. Markerstoff angereichert hat. Allerdings können, beispielsweise aufgrund ihrer geringen Größe und/oder ihres unzureichenden Kontrastes, nicht alle pathologische Gewebebereiche 7 visualisiert werden und sind dementsprechend, also aufgrund ihrer teilweisen Unsichtbarkeit für die Bedienperson, auch nicht allesamt gezielt, d.h. fokal bzw. gewebespezifisch einzeln behandelbar. Man weiß aber aus der Erfahrung, beispielsweise durch Entnahme von Zufallsbiopsien aus dem Organ 3, dass es solche pathologischen Gewebebereiche 7 dennoch fast immer gibt. Sämtliche der sichtbaren und unsichtbaren pathologischen Gewebebereiche 7 sollen nun mit Licht bestrahlt werden, um eine entsprechende therapeutische Wirkung bei allen pathologischen Gewebebereichen 7 zu erzielen. Werden bestimmte pathologischen Gewebebereiche 7, beispielsweise ein Tumor, nicht bestrahlt, so bleiben diese untherapiert und können sich vergrößern oder anderweitig medizinisch schadhaft bleiben oder werden.

In Fig. 1 ist gezeigt, dass das System 1 eine Mehrzahl von nadelartigen Lichtapplikatoren 9 aufweist, welche durch die Haut 11 des Körpers 5, also transkutan, in das Organ 3 gestochen werden. Die einzelnen Lichtapplikatoren 9 sind alle identisch zueinander und möglichst dünn ausgestaltet, um mit dem Einstich möglichst wenig gesundes Gewebe des Körpers 5 zu schädigen, d.h. um einen minimalinvasiven Eingriff zu ermöglichen. Am distalen Ende 13 eines dünnen schaftförmigen Einführabschnitts 15 des Lichtapplikators 9 ist ein lichtemittierendes Element 17 in Form einer LED angeordnet. Distalseitig von der LED 17 ist am distalen Ende 13 des Einführabschnitts 15 des Lichtapplikators 9 eine lichttransparente und lichtstreuende Applikatorspitze 19 angeordnet. Durch die Applikatorspitze 19 wird das Licht der LED 17 möglichst isotrop in einen Raumwinkel von über 3π abgestrahlt. Die Lichtabstrahlung von der Applikatorspitze 19 ist also annähernd kugelförmig, was in Fig. 1 durch eine Lichtkugel21 bzw. in der vorliegenden Schnittbilddarstellung durch einen entsprechenden Kreis angedeutet ist. Die Größe der Lichtkugel 21 als Orientierung bzw. Maß dafür, in welchem Gewebebereich bzw. Gewebevolumen eine im Hinblick auf die gewünschte therapeutische Wirkung noch ausreichend hohe Lichtmenge ankommt, hängt von der Eindringtiefe des Lichts in das Gewebe des Organs 3 ab und ist daher organspezifisch.

Das System 1 weist ferner eine nicht gezeigte Versorgungseinheit auf, mit welcher der Lichtapplikator 9 mit Strom versorgt wird, mit welchem die LED 17 betrieben wird. Die Versorgungseinheit weist vorzugsweise eine Mehrzahl von Anschlüssen für die Mehrzahl von Lichtapplikatoren 9 auf, die gleichzeitig bei der PDT zum Einsatz kommen können. Der Lichtapplikator 9 weist am proximalen Ende des Einführabschnitts 15 ein Griffelement 27 auf, mit dem eine Bedienperson den Lichtapplikator 9 manuell greifen und positionieren kann. Über ein Kabel mit einem Stecker, der in einen der Anschlüsse der Versorgungseinheit passt, kann der Lichtapplikator 9 angeschlossen und mit Strom versorgt werden (nicht abgebildet). Der Einführabschnitt 15 des Lichtapplikators 9 weist vorzugsweise einen Kern und einen vom Kern elektrisch isolierten Mantel auf, sodass Kern und Mantel als Hin- und Rückleiterpaar fungieren können, um die LED 17 mit Strom zu versorgen. Alternativ oder zusätzlich dazu kann ein extra Stromleiter im Einführabschnitts 15 des Lichtapplikators 9 vorgesehen sein.

In Fig. 1 wird anhand der Lichtkugel 21 bzw. deren Größe bereits deutlich, dass die Eindringtiefe des Lichts nicht ausreicht, um das ganze Organ 3 mit einem Lichtapplikator 9 allein zu bestrahlen. Ziel ist aber eine lückenlose Bestrahlung des ganzen Organs 3, da einerseits bei der hier vorgestellten Vorgehensweise die Bestrahlung mit Licht für gesundes Gewebe unschädlich ist, weil aufgrund der vergleichsweise geringen applizierten Lichtmenge bei der PDT eine zerstörerische Wirkung nur dort stattfinden kann, wo sich der Photosensibilisator angereichert hat, was wegen der Tumorselektivität des ausgewählten Photosensibilisators nur im malignen Gewebe passiert, und andererseits sichergestellt werden soll, dass kein pathologischer Gewebebereich 7 und insbesondere auch kein für die Bedienperson unsichtbarer pathologischer Gewebebereich 7 im Organ 3 untherapiert bleibt. Eine Bedienperson müsste also nach einer Bestrahlung den Lichtapplikator 9 an anderen Stellen neu einstechen, um mit den entsprechenden Lichtkugeln 21 nach und nach, d.h. sequentiell das gesamte Volumen des Organs 3 abzudecken.

Solch ein Vorgehen erfordert allerdings sehr gute Erfahrung der Bedienperson und ist mit vielen Risiken und Nachteilen verbunden. Zum einen kann nicht sichergestellt werden, dass am Ende das gesamte Volumen des Organs 3 lückenlos bestrahlt wurde, weil einerseits die Bedienperson viel Freiräume bei der Platzierung der Lichtapplikatoren 9 im Organ 3 hat und weil andererseits derjenige Bereich, der therapeutisch wirksam erfasst wurde, bzw. dessen Grenzen, die in Fig. 1 durch die Oberfläche der Lichtkugel 21 bzw. in der dortigen Schnittbilddarstellung durch den Umfang des Kreises dargestellt sind, nicht unmittelbar visualisiert und damit der Bedienperson zur Kontrolle nicht unmittelbar sichtbar gemacht werden können, sodass keine Gewissheit besteht, ob der Lichtapplikator 9 bzw. dessen lichtemittierende Applikatorspitze 19 immer an der richtigen Stelle platziert wurde. Zum anderen kann es zu einem "Herumstochern" kommen, bei dem zu viele Einstiche benötigt werden, die den Patienten belasten. Schließlich kann die PDT in dieser Form sehr lange dauern.

In Fig. 1 ist gezeigt, wie die PDT beschleunigt werden kann, wenn mehrere einzelne Lichtapplikatoren 9 gleichzeitig zum Einsatz kommen. Dies verteuert zwar das System 1, spart aber Zeit. Dabei ist es sinnvoll, die Applikatorspitzen 19 an sämtliche virtuelle Sollpunkte 33 einer virtuellen dreidimensionalen Sollpunktgitterstruktur 35 zu platzieren. Die Sollpunktgitterstruktur35 ist an die Form und Lage des Organs 3 sowie die Eindringtiefe des Lichts im Organ 3 angepasst. Die Sollpunktgitterstruktur 35 ist also organspezifisch. Die Abstände der virtuellen Sollpunkte 33 sind so an das Organ 3 angepasst, dass sich einerseits die Lichtkugeln 21 lückenlos überlappen und das gesamte Organ 3 lückenlos bestrahlt wird, dass aber andererseits diese Überlappung im Sinne einer patientenschonenden Vorgehensweise vorzugsweise nur so weit wie nötig vorangetrieben wird, d.h. dass unnötig kleine Abstände der einzelnen lichtemittierenden Applikatorspitzen 19 vermieden werden, um dadurch die Anzahl der Lichtapplikatoren 9 bzw. der Lichtapplikatoreinstiche zu minimieren. Aus diesen beiden gegensätzlichen Anforderungen ergibt sich eine Sollpunktgitterstruktur 35, bei der die Lage und vor allen Dingen die Abstände der virtuellen Sollpunkte 33 zueinander recht genau definiert und vergleichsweise eng toleriert sind.

Die Schwierigkeit besteht nun darin, die einzelnen Applikatorspitzen 19 genau an diesen virtuellen Sollpunkten 33 zu platzieren, da die virtuelle dreidimensionale Sollpunktgitterstruktur 35 für die Bedienperson nicht sichtbar ist und damit die virtuellen Sollpunkte 33 auch nicht gezielt und direkt von der Bedienperson mit den lichtemittierenden Applikatorspitzen 19 angesteuert werden können.

In einem ersten Schritt wird die virtuelle und unsichtbare Sollpunktgitterstruktur 35 mit ihren virtuellen und unsichtbaren Sollpunkten 33 aus dem Inneren des Körpers 5 auf eine Fixierpunktgitterstruktur 37 außerhalb des Körpers 5 abgebildet, welche durch eine real existierende und sichtbare Platzierungsschablone 39 mit real existierenden und sichtbaren und damit auch von der Bedienperson mit den lichtemittierenden Applikatorspitzen 19 gezielt und direkt ansteuerbaren Fixierpunktaufnahmen 51 gebildet wird. Bei dieser Abbildung der Sollpunktgitterstruktur 35 auf die Fixierpunktgitterstruktur 37 handelt es sich um eine Parallelverschiebung bzw. Translation um eine definierte Länge d, die durch den Abstand des Organs 3 zum Raum außerhalb des Körpers, wo die Platzierungsschablone 39 positioniert werden soll, definiert ist, parallel zu einer Achse E, deren Richtung durch die Einstichachse der Applikatoren 9 definiert ist. Die Achse E verläuft vorzugsweise aber nicht zwangsweise orthogonal zur Haut 11 des Patienten.

Erst in einem späteren Schritt erfolgt dann die eigentliche, therapievorbereitende Applikatorplatzierung, indem die einzelnen lichtemittierenden Applikatorspitzen 19 zunächst zu den real existierenden und sichtbaren und damit gezielt und direkt ansteuerbaren Fixierpunktaufnahmen 51 der Platzierungsschablone 39 geführt werden, um von dort in definierter, eindeutiger und damit sicherer Weise in bzw. zu den virtuellen und für die Bedienperson unsichtbaren Sollpunkten 33 "abgebildet" bzw. bewegt zu werden. Diese im Rahmen der zweistufigen Vorgehensweise durchzuführende zweite Abbildung, also die Parallelverschiebung der lichtemittierenden Applikatorspitzen 19 von außerhalb des Körpers 5 in den Körper 5 hinein, ist nun aber entsprechend dieser Vorgehensweise gerade dadurch gekennzeichnet, dass sie zur Parallelverschiebung der Sollpunktgitterstruktur 35 von innerhalb des Körpers 5 nach draußen invers ist. Konkret heißt das, dass die Applikatorspitzen 19 - ausgehend von den Fixierpunktaufnahmen 51 und damit ausgehend von den Zielpunkten der ersten Abbildung bzw. Parallelverschiebung - um die gleiche definierte Länge d und parallel zur gleichen Achse E, abergenau entgegen der ersten Parallelverschiebung bewegt werden.

Dabei wird die Definiertheit, Eindeutigkeit und Sicherheit hinsichtlich der Zielgenauigkeit bei der Platzierung der einzelnen lichtemittierenden Applikatorspitzen 19 in den Sollpunkten 33 im Organ 3, also die hier entscheidenden Merkmale im Hinblick auf die geforderte lückenlose Bestrahlung des Organs 3, beim Einführen der lichtemittierenden Applikatorspitzen 19 von den Fixierpunktaufnahmen 51 außerhalb des Körpers 5 ausgehend zu den virtuellen Sollpunkten 33 innerhalb des Körpers 5 - trotz der Unsichtbarkeit der Sollpunkte 33 für die Bedienperson und der daraus resultierenden Unmöglichkeit, diese gezielt ansteuern zu können - dadurch erreicht, dass die Freiheitsgrade für die Bedienperson bei der Bewegung der Lichtapplikatoren 9 bzw. bei der Platzierung der lichtemittierenden Applikatorspitzen 19 im Körper 5 auf ein Minimum reduziert werden, d.h. dass es keine andere Bewegungsmöglichkeit gibt als die erforderliche, zur ersten Parallelverschiebung der Sollpunktgitterstruktur 35 exakt inverse Parallelverschiebung der lichtemittierenden Applikatorspitzen 19.

Das Führen der Bewegung der einzelnen lichtemittierenden Applikatorspitzen 19 ausschließlich gemäß der inversen Parallelverschiebung in den Körper 5 hinein, geschieht erstens dadurch, dass die Platzierungsschablone 39 neben den schon genannten Fixierpunktaufnahmen 51 zusätzlich mit Führungen 41 versehen ist, welche eine definierte Ausrichtung haben, nämlich eine parallele Ausrichtung zur Achse E, wodurch nur eine einzige, nämlich die für die gewünschte inverse Parallelverschiebung definierte Bewegungsrichtung der Lichtapplikatoren 9 mit ihren Applikatorspitzen 19, parallel zur Achse E, zugelassen bzw. möglich wird, und zweitens dadurch, dass die Lichtapplikatoren 9 allesamt mit mindestens einem Fixierpunkt 43 versehen sind, welcher einen definierten Abstand d zur lichtemittierenden Applikatorspitze 19 hat, der nämlich genau der Verschiebungslänge d der Sollpunktgitterstruktur 35 bei der ersten Abbildung bzw. Parallelverschiebung entspricht, und welcher in bzw. an den Fixierpunktaufnahmen 51 der Platzierungsschablone 39 fixierbar ist, wodurch nur eine einzige, nämlich die für die gewünschte inverse Parallelverschiebung definierte Eindringtiefe der zugehörigen lichtemittierenden Applikatorspitze 19 in den Körper 5 und in das Organ 3 zugelassen bzw. möglich wird.

Beides zusammen führt also dazu, dass bei einer Bewegung der einzelnen Lichtapplikatoren 9 in distale Richtung bis auf Anschlag in der Platzierungsschablone 39 - ausgehend von den sichtbaren und deshalb direkt ansteuerbaren Fixierpunktaufnahmen 51 - die lichtemittierenden Applikatorspitzen 19 auch allesamt automatisch in den Sollpunkten 33 ankommen und damit auch automatisch die gewünschte lückenlose Bestrahlung des Organs 3 gewährleistet ist, vorausgesetzt, dass auch alle Fixierpunktaufnahmen 51 der Schablone 39 mit Lichtapplikatoren 9 belegt und aktiviert sind.

Die Bedienperson muss also nur die einzelnen Lichtapplikatoren 9 an die Fixierpunktaufnahmen 51 heranführen, dann durch die Führungen 41 hindurchführen und transkutan in das Organ 3 einstechen bis sich der Fixierpunkt 43 am Gitterpunkt der Fixierpunktgitterstruktur 37, d.h. an der Fixierpunktaufnahme 51, befindet. Dann ist auch die Applikatorspitze 19 automatisch am zugehörigen Sollpunkt 33 platziert. Die Bedienperson hat dadurch keine oder nur wenige Freiheitsgrade und benötigt keine große Erfahrung. Außerdem ist die Platzierung schnell durchführbar mit gerade so vielen Einstichen wie nötig, aber mit so wenig Einstichen wie möglich. Die PDT kann zeitgleich mit sämtlichen platzierten Lichtapplikatoren 9 durchgeführt werden, was eine kurze Behandlungsdauer ermöglicht.

Diese Vorgehensweise basiert also erstens auf der Durchführung zweier Abbildungen, nämlich einerseits der Abbildung der Sollpunkte 33 auf die Fixierpunktaufnahmen 51 der Platzierungsschablone 39 und andererseits der Abbildung der lichtemittierenden Applikatorspitzen 19 von den Fixierpunktaufnahmen 51 ausgehend zurück auf die Sollpunkte 33, wobei die zweite Abbildung dementsprechend dadurch gekennzeichnet ist, dass sie zur ersten Abbildung invers ist, und zweitens darauf, dass mechanisch-konstruktiv sichergestellt ist, dass die zweite Abbildung, welche der eigentlichen Applikatorplatzierung durch die Bedienperson entspricht, alternativlos ist, d.h. eine von der zweiten Abbildung abweichende Bewegung der Applikatoren 9 bzw. der Applikatorspitzen 19 - von den Fixierpunkten bzw. Fixierpunktaufnahmen 51 ausgehend in den Körper 5 und in das Organ 3 hinein - gar nicht möglich ist.

Mit zunehmender Größe des Organs 3 wächst die Anzahl der erforderlichen Lichtapplikatoren 9 sehr stark an. Beispielsweise bedeutet die Verdopplung des Durchmessers eines kugelförmigen Organs 3, dass achtmal so viele Lichtapplikatoren 9 benötigt werden. Wenn außerdem einerseits die Eindringtiefe des Lichtes vergleichsweise gering ist und andererseits die Ausdehnung des Organs 3 entlang der Einstichachse E vergleichsweise groß ist, dann ist die Dichte der Lichtapplikatoren 9 in den Ebenen senkrecht zur Einstichachse E entsprechend hoch und es kann passieren, dass die Lichtapplikatoren 9 nicht mehr genügend Platz nebeneinander finden.

Diesem Problem kann mit der Durchführung einer schrittweisen PDT begegnet werden. In Fig. 1 ist gezeigt wie die PDT schrittweise bzw. nacheinander in den Sollpunktgitterflächen 45, dann 46, usw. der Sollpunktgitterstruktur 35 durchgeführt werden kann. Dazu werden die Fixierpunkte 43 der Lichtapplikatoren 9 an den Gitterpunkten bzw. den Fixierpunktaufnahmen 51 einer zugehörigen Fixierpunktgitterfläche 53 der Fixierpunktgitterstruktur 37 platziert bzw. fixiert, sodass die einzelnen Applikatorspitzen 19 an den zugehörigen Sollpunkten 33 der Sollpunktgitterfläche 45 platziert sind. Die PDT wird (in Fig. 1 beispielhaft) erst für die ersten beiden Sollpunktgitterflächen 45 durchgeführt, die vorzugsweise die am tiefsten unter der Haut liegenden Sollpunktgitterflächen 45 sind, also am weitesten distalwärts liegen. Dann können die Lichtapplikatoren 9 um einen Abstand 2k proximalwärts zurückgezogen werden, um die PDT für die nächsten beiden Sollpunktgitterflächen 46 durchzuführen. Dies wird schrittweise für alle Sollpunktgitterflächenpaare 45, 46, usw. durchgeführt bis das gesamte Volumen des Organs 3 mit Licht bestrahlt wurde. In Fig. 1 definiert die Platzierungsschablone 39 also beispielhaft gleich zwei zueinander parallel im Abstand k versetzte Fixierpunktgitterflächen 53, sodass zwei benachbarte Sollpunktgitterflächen 45 gleichzeitig bestrahlt werden können. Dann können die Lichtapplikatoren 9 um einen Abstand 2k der Sollpunktgitterflächen 45 proximalwärts zurückgezogen werden, um die PDT für die nächsten zwei Sollpunktgitterflächen 45 durchzuführen. Vorteilhaft bei dieser schrittweisen Vorgehensweise ist, dass weniger Lichtapplikatoren 9 und weniger Einstiche benötigt werden sowie die Behandlungszeit verkürzt wird.

In Fig. 1 ist die Platzierungsschablone 39 als ganze gegenüber einer festen Referenzfläche 57 um den Abstand 2k definiert verschiebbar. Dazu definiert die Platzierungsschablone 39 mittels der Befestigungsvorrichtung 55 mehrere im Abstand 2k angeordnete Fixierpunkte, die als Eingriffsaufnahmen 69 in der Befestigungsvorrichtung 55 ausgebildet sind, um die Fixierpunktaufnahmegitterflächen 53 als ganze um den Abstand 2k definiert verschieben zu können. Dies hat den Vorteil, dass die Lichtapplikatoren 9 beim Wechsel zu den nächsten Sollpunktgitterflächen 46 nicht einzeln bewegt werden müssen, sondern zusammen konzertiert bewegt werden. Dies beschleunigt und vereinfacht den Behandlungsprozess. Nach der PDT mit den Applikatorspitzen 19 in der ersten und zweiten Sollpunktgitterfläche 45 wird das Organ 3 mit den Applikatorspitzen 19 in der dritten und vierten Sollpunktgitterfläche 46 bestrahlt.

Die Platzierungsschablone 39 ist in Fig. 1 ein Block oder anders geformter Körper mit einer gewissen Dicke entlang der Einstichachse E, durch welchen eine Vielzahl von parallel zueinander angeordneten Führungen 41 entlang der Einstichachse E verläuft. Jede Führung 41 weist eine Fixierpunktaufnahme 51 auf, welche einen Gitterpunkt der Fixierpunktgitterstruktur 37 bildet. Die so gebildete dreidimensionale Fixierpunktgitterstruktur 37 ist identisch mit der organspezifischen virtuellen Sollpunktgitterstruktur 35 für die Applikatorspitzen 19 und geht aus jener durch eine Parallelverschiebung bzw. Translation um die Länge d hervor. Jeder Lichtapplikator 9 weist einen dünnen Einführabschnitt 15 auf und einen dickeren proximalen Abschnitt, der als Griffelement 27 sowie der Führung in den Führungen 41 dient. Die Führungen 41 sind entsprechend dem Durchmesser des Einführabschnitts 15 und des dickeren proximalen Abschnitts der Lichtapplikatoren 9 angepasst dimensioniert. Die Lichtapplikatoren 9 sind alle identisch, um das Risiko einer fehlerhaften Auswahl eines "falschen" Lichtapplikators 9 und daraus resultierend eine Fehlpositionierung der lichtemittierenden Applikatorspitze 19 im Organ 3 zu vermeiden. Die Bedienperson hat hier keine Freiheitsgrade, sondern kann die einzelnen Lichtapplikatoren 9 nur auf eine Art und Weise einstechen bis die Fixierpunkte 43 in den Fixierpunktaufnahmen 51 einrasten. Dazu können die Fixierpunktaufnahmen 51 Maulklammern oder, wie in Fig. 1 gezeigt, Rastnasen oder ein anderweitiges Klemmmittel aufweisen. Die Fixierpunkte 43 und die Fixierpunktaufnahmen 51 bilden in Fig. 1 beispielhaft eine Art Bajonettverschluss, bei dem ein lateraler Vorsprung 63 am Lichtapplikator 9 durch Drehung des Lichtapplikators 9 um die Einstichachse E in eine entsprechende laterale Aufnahmesicherung 65 in der Führung 41 drehbar ist. Dies gibt der Bedienperson ein haptisches Feedback zur korrekten Platzierung und verhindert ein ungewolltes proximalwärtiges Verrutschen der Lichtapplikatoren 9. Die Platzierungsschablone 39 ist entlang der Einstichachse E mittels ihrer Befestigungsvorrichtung 55 geführt bewegbar und relativ zur Referenzfläche 57 fixierbar. Die diesbezügliche Position der Platzierungsschablone 39 kann vorzugsweise wie folgt bestimmt werden. Die Platzierungsschablone 39 wird mit der Befestigungsvorrichtung 55 an der Referenzfläche 57 zunächst ganz nahe am Körper 5 bzw. an der Haut 11 platziert und fixiert. Anschließend wird vorzugsweise ein bzgl. einer Ebene senkrecht zur Einstichachse E zentral und bzgl. der Einstichachse E selbst distal zu positionierender, erster Lichtapplikator 10 eingestochen und - mit einem bildgebenden Verfahren (beispielsweise Ultraschall-Sonografie) kontrolliert - soweit vorgeschoben, bis seine Applikatorspitze 19 den distalen Bereich des Organs 3 erreicht hat. Dann wird - unter Aufrechterhaltung dieser erreichten Position des ersten Lichtapplikators 10 bzw. dessen Applikatorspitze 19 - die Platzierungsschablone 39 mittels der Befestigungsvorrichtung 55 an der Referenzfläche 57 axial, d.h. entlang der Einstichachse E, soweit in proximale Richtung von der Haut 11 weg zurückgezogen, bis der Fixierpunkt 43 in der Fixierpunktaufnahme 51 fixiert werden kann. In dieser Position wird dann die Platzierungsschablone 39 mittels der Befestigungsvorrichtung 55 an der Referenzfläche 57 fixiert.

Mit diesem Vorgang wird der definierte Abstand d zwischen der virtuellen Sollpunktgitterstruktur 35 und Fixierpunktgitterstruktur 37 hergestellt. Dieser Vorgang vollendet damit den ersten Schritt der hier angewandten Vorgehensweise, nämlich die erste Abbildung, d.h. die Parallelverschiebung bzw. Translation der virtuellen Sollpunktgitterstruktur 35 im Organ 3 innerhalb des Körpers 5 in einen Bereich außerhalb des Körpers 5, und schafft so die Voraussetzung für die eigentliche Therapievorbereitung, nämlich die korrekte Platzierung der restlichen Lichtapplikatoren 9 bzw. deren lichtemittierender Applikatorspitzen 19 im Organ 3.

Die restlichen Lichtapplikatoren 9 müssen dann nur noch durch die verbleibenden Führungen 41 der Platzierungsschablone 39 hindurchgeführt und mit ihren Fixierpunkten 43 an der jeweiligen Fixierpunktaufnahme 51 fixiert werden. Die Freiheitsgrade der Bedienperson sind dabei minimiert, was wiederum bedeutet, dass die Positionen der restlichen Lichtapplikatoren 9 und in der Folge die Positionen ihrer lichtemittierenden Applikatorspitzen 19 nach der Fixierung der Platzierungsschablone 39 eindeutig festgelegt sind und dadurch automatisch mit den Sollpunkten 33 übereinstimmen.

Das in Fig. 1 gezeigte Prinzip, mehrere Sollpunktgitterflächen 45 gleichzeitig zu behandeln, gewinnt vor allen Dingen dann an Bedeutung, wenn die Ausdehnung des Organs 3 - vor allen Dingen auch entlang der Einstichachse E - vergleichsweise groß ist, weil dadurch in der gleichen Zeit bzw. mit dergleichen Anzahl an sequenziellen Bestrahlungseinheiten (im vorliegenden Beispiel drei Bestrahlungseinheiten) ein deutlich größeres Volumen behandelt werden kann. Die Platzierungsschablone 39 weist dazu Fixierpunktaufnahmen 51 von zwei benachbarten Fixierpunktaufnahmegitterflächen 53 auf. Die Platzierungsschablone 39 ist dann um die doppelte Länge, nämlich um 2k verschiebbar, um die PDT für die folgenden zwei Sollpunktgitterflächen 45 durchzuführen. Mit dieser Vorgehensweise, d.h. dass in einer Bestrahlungseinheit alle Sollpunkte aus mehreren Sollpunktgitterflächen 45 gleichzeitig abgedeckt werden, können in der gleichen Zeit, d.h. mit der gleichen Anzahl an Bestrahlungseinheiten, größere d.h. auch stärker entlang der Einstichachse E ausgedehnte Organe 3 behandelt werden. Die Befestigungsvorrichtung 55 der Platzierungsschablone 39 weist ein Eingriffselement 67 auf, das in fest bezüglich der Referenzfläche 57 jeweils im Abstand 2k entlang der Einstichachse E angeordnete Eingriffsaufnahmen 69 eingreift.

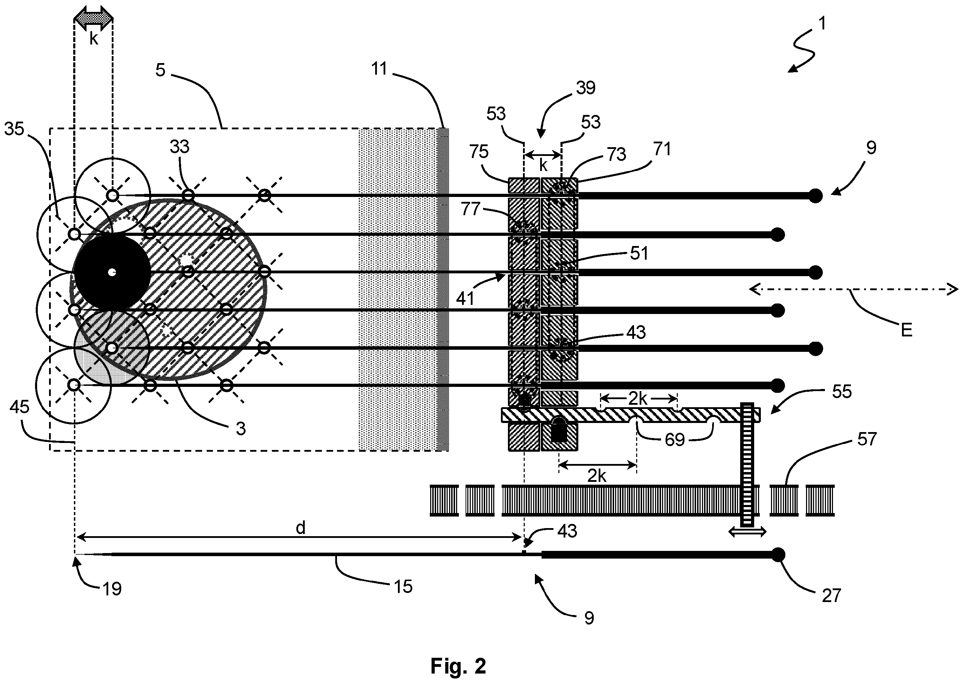

Fig. 2 zeigt ein Lösungsprinzip für das "Aufgabelungsproblem"-Problem, bei dem die in das Organ 3 eingestochenen einzelnen Lichtapplikatoren 9 beim proximalwärtigen Zurückziehen das Organ 3 proximalwärts zurückziehen und/oder das Organ 3 verformen bzw. dehnen/strecken. Die Platzierungsschablone 39 weist hier nämlich mindestens zwei Teile auf, nämlich einen ersten Schablonenteil 71, der eine erste Untergruppe 73 der Führungen 41 mit Fixierpunktaufnahmen 51 definiert, und einen zweiten Schablonenteil 75, der eine zweite Untergruppe 77 der Führungen 41 mit Fixierpunktaufnahmen 51 definiert. Der erste Schablonenteil 71 ist dabei relativ zum zweiten Schablonenteil 75 entlang der Einstichachse E geführt verschiebbar. Mittels der Schablonenteile 71, 75 können verschiedene Untergruppen von Lichtapplikatoren 9 durch die entsprechenden Untergruppen 73, 77 von Führungen 41 mit Fixierpunktaufnahmen 51 gemeinsam konzertiert wahlweise bewegt oder festgehalten werden. Dies hat den Vorteil, dass mit einer Untergruppe von Lichtapplikatoren 9 das Organ 3 festgehalten werden kann, während die andere Untergruppe bewegt wird. Der "Aufgabeleffekt" kann also dadurch vermieden oder zumindest verringert werden, dass eine Untergruppe von Lichtapplikatoren 9 festgehalten wird während die andere herausgezogen wird. Die jeweiligen Untergruppen können abwechselnd bewegt werden während die andere festgehalten wird. Die gemeinsame Bewegung und das gemeinsame Festhalten der Lichtapplikatoren 9 kann durch entsprechende Bewegung bzw. Festhalten der Schablonenteile 71, 75 bewirkt werden. Der erste Schablonenteil 71 mit der ersten Untergruppe von Lichtapplikatoren 9 kann proximalwärts bewegt werden während der zweite Schablonenteil 75 festgehalten wird. Die Summe der Haftreibungskräfte, welche sowohl die bewegten Lichtapplikatoren 9 der ersten Untergruppe 73 als auch die festgehaltenen Lichtapplikatoren 9 der zweiten Untergruppe 77 auf das Organ 3 ausüben, ergibt in erster Näherung Null. Dadurch wird es mit dieser Vorgehensweise möglich, eine Vielzahl von Lichtapplikatoren 9 gemeinsam bzw. gleichzeitig in eine gemeinsame Richtung, hier in proximale Richtung, zu bewegen, ohne dabei das Organ 3 mitzunehmen und/oder ohne das Organ 3 zu verformen.

Der zweite Schablonenteil 75 mit der zweiten Untergruppe 77 von Lichtapplikatoren 9 kann proximalwärts nachgezogen werden während der erste Schablonenteil 71 mit der ersten Untergruppe 73 von Lichtapplikatoren 9 festgehalten wird. Die Summe aller Haftreibungskräfte, welche von allen Lichtapplikatoren 9 gemeinsam auf das Organ 3 ausgeübt wird, ergibt auch hier wieder in erster Näherung Null, sodass das Organ 3 trotz der gleichzeitigen Bewegung einer Vielzahl von Lichtapplikatoren 9, nämlich derjenigen der zweiten Untergruppe 77, in eine gemeinsame Richtung, weder mitgezogen noch verformt wird.

Die vergleichsweise hohe Dichte der Führungen 41 und Fixierpunktaufnahmen 51 und daraus resultierend die entsprechend hohe potenzielle Dichte von Applikatoren 9 im Organ 3, sowie die Anordnung der Führungen 41 und Fixierpunktaufnahmen 51 unterschiedlicher Untergruppen 73 und 77 und entsprechend der Applikatoren 9 unterschiedlicher Untergruppen 73 und 77 zueinander, d.h. der regelmäßige Wechsel der Zugehörigkeit von Führungen 41 und Fixierpunktaufnahmen 51 zu den unterschiedlichen Untergruppen 73 und 77, führen dazu, dass auch lokale Verschiebungen und Verformungen innerhalb des Organs 3 weitgehend vermieden werden können.

Die Platzierungsschablone 39 weist in Fig. 2 zwei parallele Platten 71, 75 auf, welche die Schablonenteile 71, 75 bilden und entlang der Einstichachse E geführt relativ zueinander beweglich sind. Der erste Schablonenteil 71 weist die Fixierpunktaufnahmen 51 von einer ersten Fixierpunktaufnahmegitterfläche 53 auf und der zweite Schablonenteil 75 weist die Fixierpunktaufnahmen 51 von einer zweiten Fixierpunktaufnahmegitterfläche 53 auf.

Ist ein "Aufgabeleffekt" zu befürchten, kann zunächst der erste Schablonenteil 71 um 2k zurückgezogen werden, während der zweite Schablonenteil 75 festgehalten wird. Sobald der erste Schablonenteil 71 positioniert ist, kann der zweite Schablonenteil 75 um 2k proximalwärts nachgezogen werden während der erste Schablonenteil 71 festgehalten wird.

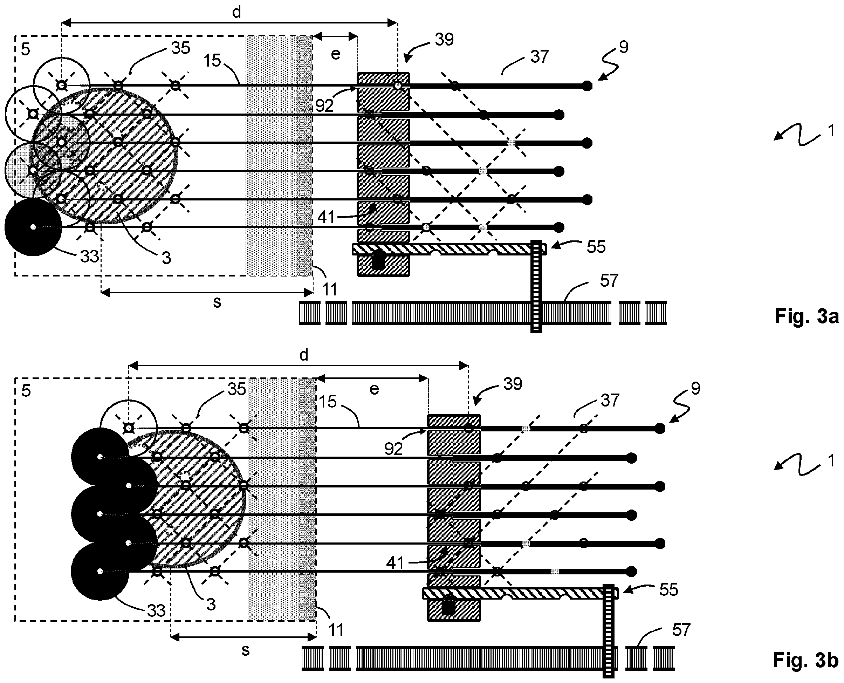

Fig. 3a,b zeigen das in Fig. 1 gezeigte System in verschiedenen Anwendungsfällen. In Fig. 3a liegt das Organ 3 relativ weit entfernt von der Haut 11 des Körpers 5 des Patienten. In Fig. 3b liegt das Organ 3 näher an der Haut 11 des Körpers 5 des Patienten. Da man für die verschiedenen Anwendungsfälle nicht unterschiedlich lange Versionen von Lichtapplikatoren 9 bevorraten und entsprechend korrekt auswählen möchte, ist ein Abstand e zwischen Haut 11 und Platzierungsschablone 39 so einzustellen, dass die Fixierpunktgitterstruktur 37 korrekt platziert ist, damit die diesbezüglich um den Abstand d parallelverschobene Sollpunktgitterstruktur 35 korrekt im Organ 3 liegt. Dies wird wie bereits oben beschrieben über eine entsprechende Positionierung der Befestigungsvorrichtung 55 gegenüber der mit dem Körper 11 verbundenen Referenzfläche 57 erreicht. Der Körper 11 kann beispielsweise auf einem Behandlungstisch fixiert sein, der die Referenzfläche 57 definiert.

Der Abstand e kann, wie in Fig. 3b gezeigt, insbesondere dann relativ groß sein, wenn das Organ 3 relativ nah an der Haut 11 liegt. Der durch die Lichtapplikatoren 9 festgelegte Abstand d ist nämlich für maximal tief unter der Haut 11 liegende Organe 3 ausgelegt, siehe dazu Fig. 3a, um auch diese mit den gleichen Lichtapplikatoren 9 noch erreichen und behandeln zu können. Folglich ergibt sich ein entsprechend großer Abstand e bei näher an der Haut 11 befindlichen Organen 3 wie in Fig. 3b dargestellt.

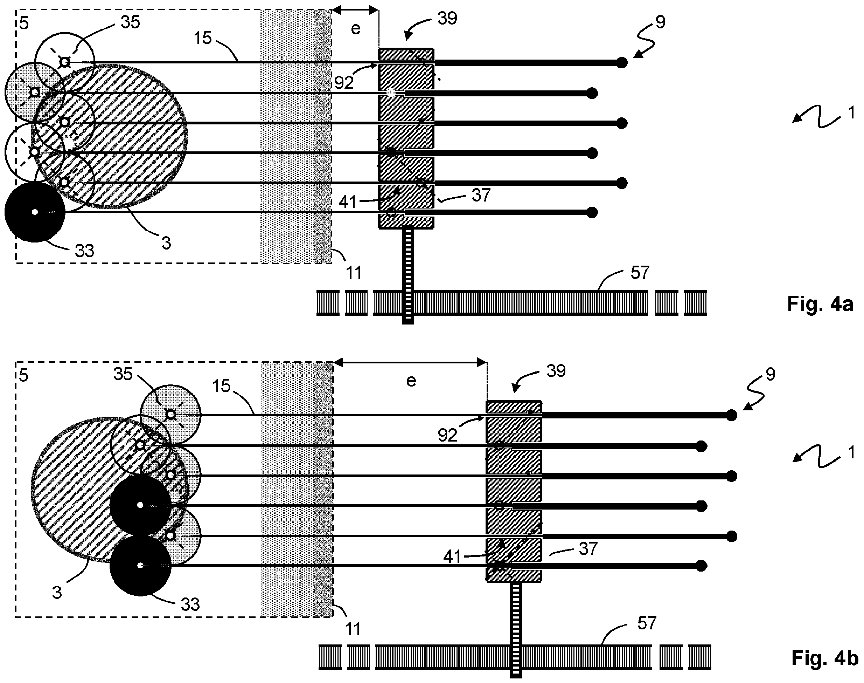

In Fig. 4a,b wird deutlich, dass sich ein größerer Abstand e nicht nur in verschiedenen Anwendungsfällen ergeben kann, sondern auch während eines Anwendungsfalls. Dies ist nämlich dann der Fall, wenn das Organ 3 eine gewisse Länge entlang der Achse E hat. Nach jedem Bestrahlungsschritt in einer oder mehr Sollpunktgitterflächen 45 wird ja die Platzierungsschablone proximalwärts bewegt, sodass sich der Abstand e nach und nach vergrößert. Erstreckt sich das Organ 3 also bis relativ nah an die Haut 11, so wird der Abstand e für die Behandlung des proximalen Endbereichs des Organs 3 relativ groß. Falls man beispielsweise im proximalen Organbereich eine höhere Applikatorendichte benötigt, weil dort beispielsweise mehr pathologisches Gewebe 6 ist oder vermutet wird, müssten bei relativ großem Abstand e Lichtapplikatoren nachgestochen werden.

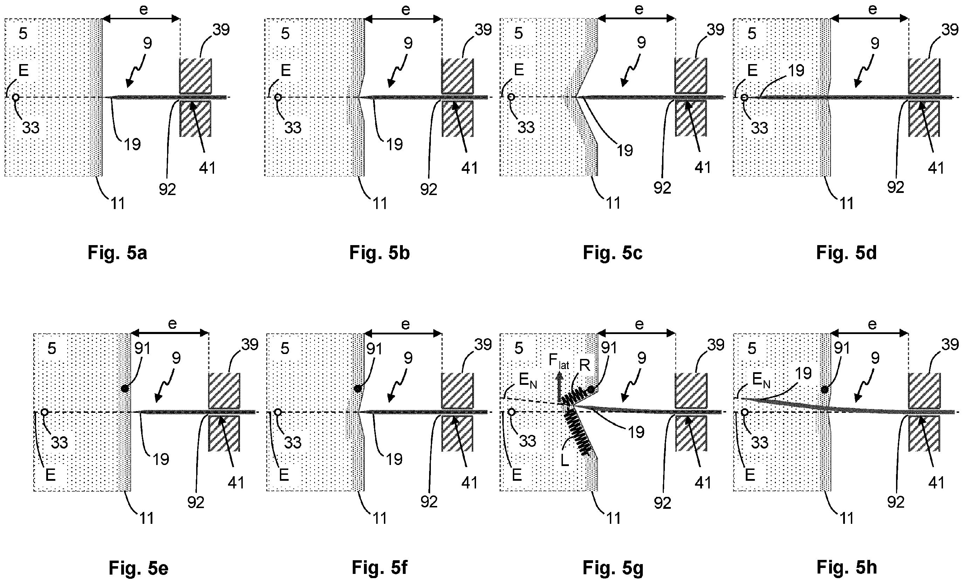

Fig. 5a-h zeigen eine Problematik beim Hauteinstich für den Fall, dass der Abstand e zwischen der Haut 11 und einem distalen Ende 92 der Führung 41 der Platzierungsschablone 39 relativ groß wird, beispielsweise wie in Fig. 3b und 4b. In Fig. 5a-d ist ein problemloser Haueinstich gezeigt, bei dem sich die Applikatorspitze 19 genau entlang der Einstichachse E bewegt und zielgenau den Sollpunkt 33 im Organ 3 erreicht. Das liegt u.a. daran, dass sowohl die Haut 11 als auch das nachfolgend durchstochene Gewebe im Körper 5 homogen strukturiert sind. Dadurch kann beim Einführen des Lichtapplikators 9 durch die Haut 11 in den Körper 5 in axialer Richtung auch nur eine solche resultierende Kraft auf den Lichtapplikator 9 zurückwirken, die ausschließlich eine axiale Komponente, jedoch keine seitliche bzw. laterale Komponente Fₗₐₜ aufweist.

In Fig. 5e-h weist die Haut 11 allerdings eine Inhomogenität 91 im strukturellen Aufbau des Gewebes auf. Diese könnte durch eine Narbe, Knochen- oder Knorpelnähe, eine Verwachsung, eine generell veränderte Faserdichte oder eine anderweitige Störung hinsichtlich der Homogenität verursacht sein. Wie in den Fig. 5a-d wirkt auch hier auf den Lichtapplikator 9, der die Haut 11 durchdringt und in den Körper 5 eindringt und dabei eine Kraft auf das Gewebe ausübt, eine Kraft F zurück. Wie in Fig. 5g in einem Federmodell verdeutlicht, handelt es sich aber in diesem Fall aufgrund der Inhomogenität 91 um eine resultierende Kraft, die auch eine seitliche bzw. laterale Komponente Fₗₐₜ aufweist. Diese seitliche bzw. laterale Kraft Fₗₐₜ wirkt als Biegekraft auf den Lichtapplikator 9 und kann dadurch eine Verbiegung desselben verursachen. Entscheidend für das Maß bzw. die Stärke der resultierenden Lichtapplikator-Verbiegung ist das Biegemoment M, welches durch das Produkt aus Biegekraft, also der lateralen Kraftkomponente Fₗₐₜ der auf den Lichtapplikator 9 zurückwirkenden resultierenden Kraft, und überstehender Lichtapplikatorlänge, d.h. dem Abstand e zwischen Haut 11 und Platzierungsschablone 39 gebildet wird. Das wiederum bedeutet jedoch erstens, dass mit zunehmendem Abstand e das Maß bzw. die Stärke der resultierenden Lichtapplikator-Verbiegung immer weiter zunimmt. Das bedeutet zweitens, dass auch schon eine kleine laterale Kraft Fₗₐₜ auf den Lichtapplikator 9 bzw. dessen Applikatorspitze 19 eine vergleichsweise starke Verbiegung des Lichtapplikators 9 bewirken kann, wenn nur der Abstand e genügend groß ist, wie in Fig. 5g, h verdeutlicht. Ganz konkret bedeutet das aber, dass bereits geringe und damit scheinbar unbedeutende Inhomogenitäten 91 in der Gewebestruktur im Bereich der Haut 11 ganz wesentlich zu einer Verbiegung des Lichtapplikators 9 beitragen können, nämlich dann, wenn sich die Platzierungsschablone 39 nicht in unmittelbarer Nähe des Körpers 5 bzw. der Haut 11 befindet. Ist der Lichtapplikator 9 außerdem im Sinne der Schonung des Patienten noch sehr dünn ausgeführt, dann ist die Verbiegung entsprechend noch stärker bzw. kommt auch schon bei entsprechend kürzeren Abständen e zum Tragen. Solch eine Verbiegung führt allerdings dazu, dass sich die Applikatorspitze 19 nicht mehr entlang der gewünschten Einstichachse E bewegt, sondern entlang einer anderen Achse E_{N}, welche eben nicht mehr zum Sollpunkt 33 führt. Die lichtemittierende Applikatorspitze 19 läge dann, nach abgeschlossener Platzierung des Applikators 9, nicht mehr wie geplant auf dem Sollpunkt 33 und bei der Bestrahlung könnten Organbereiche entsprechend unbestrahlt oder zumindest unzureichend bestrahlt bleiben.

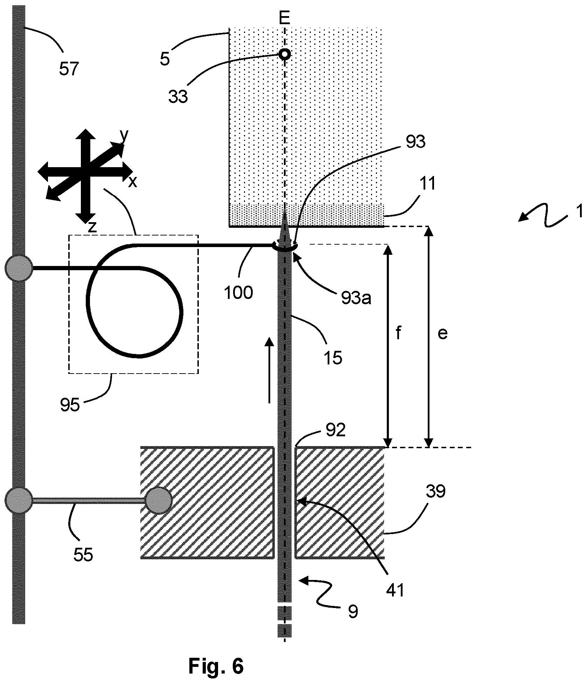

Figuren 6 bis 12 zeigen sowohl schematische Darstellungen der prinzipiellen Vorgehensweise als auch konkrete Ausführungsformen der erfindungsgemäßen Lösung für dieses Problem. Ein zwischen der Haut 11 und der Platzierungsschablone 39 verstellbarer und damit weitgehend beliebig positionierbarer Lagerpunkt 93 für den Lichtapplikator 9 verkürzt nämlich den für die Verbiegung wirksamen Abstand e um einen Abstand f des Lagerpunkts 93 von der Platzierungsschablone 39. Dadurch wird es möglich, ein auf die Applikatorspitze 19 potenziell wirkendes Biegemoment (bedingt durch mindestens eine Inhomogenität 91 im Bereich der Haut 11), welches die entscheidende Größe ist für das Maß bzw. die Stärke der Verbiegung des Lichtapplikators 9 beim Einführen desselben und welches damit die Zielgenauigkeit der Applikatorspitze 19 gegenüber dem zu erreichenden Sollpunkt 33 bei der Applikatorplatzierung im Wesentlichen bestimmt, entscheidend zu reduzieren bzw. zu minimieren. Der Lagerpunkt 93 ist hier durch eine Öffnung 93a in einem Lagerpunktelement 100 gebildet, durch welche der jeweilige Lichtapplikator 9 passgenau durchsteckbar ist. Das Lagerpunktelement 100 definiert also für jeden der Lichtapplikatoren 9 einen mit der Einstichachse E koaxial positionierten oder positionierbaren Lagerpunkt 93 zum lateralen Lagern des jeweiligen Lichtapplikators 9, wobei der distale Abstand f des Lagerpunkts 93 von der Platzierungsschablone 39 einstellbar ist und zum Zwecke der Minimierung eines potenziellen Biegemoments auf die Lichtapplikatorspitze 19 beim Einführen des Lichtapplikators 9 in den Körper 5 maximiert werden kann.

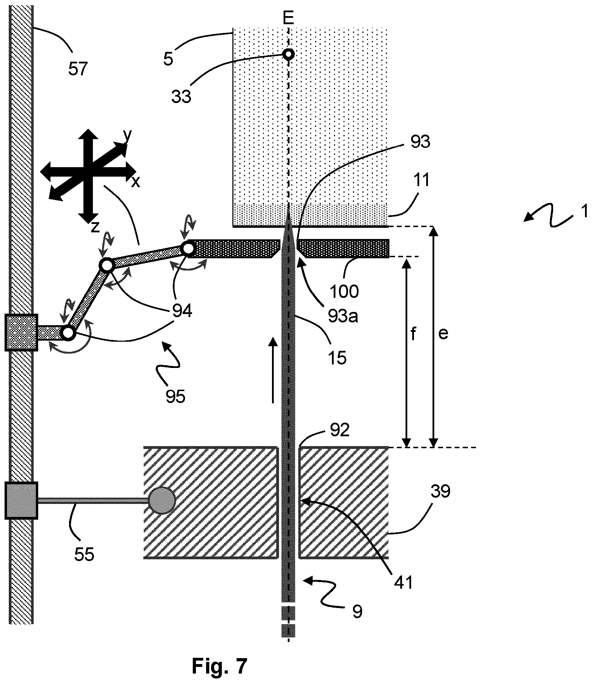

Kennzeichnend für die in Fig. 6 dargestellte prinzipielle Vorgehensweise und ihrem in Fig. 7 gezeigten konkreten Ausführungsbeispiel ist, dass das Lagerpunktelement 100 nicht nur entlang der Einstichachse E relativ zur Platzierungsschablone 39 beweglich gelagert und positioniert arretierbar ist, sondern auch in einer sich dazu quer erstreckenden xy-Ebene. Dazu ist das Lagerpunktelement 100 mittels einer in drei Raumrichtungen x,y,z über Gelenke 94 verstellbaren Verstellkomponente 95, welches in der Prinzipdarstellung der Fig. 6 in schematischer und symbolischer Weise als Flexarm bzw. Schwanenhals dargestellt ist, mit der Referenzfläche 57 verbunden. Der Lagerpunkt 93 des Lagerpunktelements 100 kann dann in der xy-Ebene fluchtend mit der Führung 41 der Platzierungsschablone 39 positioniert und arretiert werden. In z-Richtung, also entlang der Einstichachse E, kann ein möglichst großer Abstand f eingestellt werden, damit der Lichtapplikator 9 durch Minimierung eines potenziell wirksamen Biegemoments auf die Applikatorspitze 19 - bedingt durch potenzielle Inhomogenitäten 91 im Bereich der Haut 11 - bestmöglich gegen Verbiegung beim Einstich in die Haut 11 gesichert ist.

Fig. 7 zeigt ein konkretes Ausführungsbeispiel der in Fig. 6 dargestellten prinzipiellen Vorgehensweise. Dabei ist die Verstellkomponente 95 als Gelenkarm mit Gelenken 94 ausgeführt, die im Sinne einer maximalen Bewegungsfreiheit zumindest zum Teil oder alle als Kugelgelenke realisiert sein können. Das Lagerpunktelement 100 ist als Lochplatte ausgeführt, welche mit dem Gelenkarm 95 direkt verbunden ist. Die Öffnung 93a ist als Loch in der Lochplatte ausgeführt, welches zusätzlich mit einer Einführschräge versehen ist, damit der Lichtapplikator 9 besser durchgeführt werden kann.

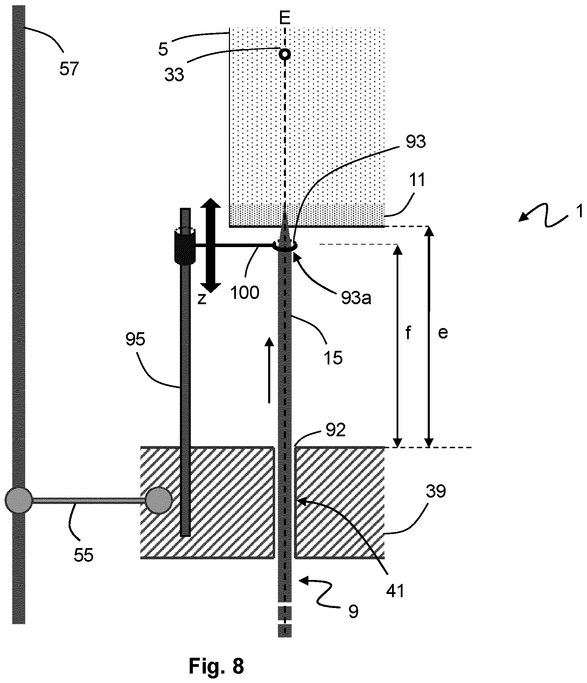

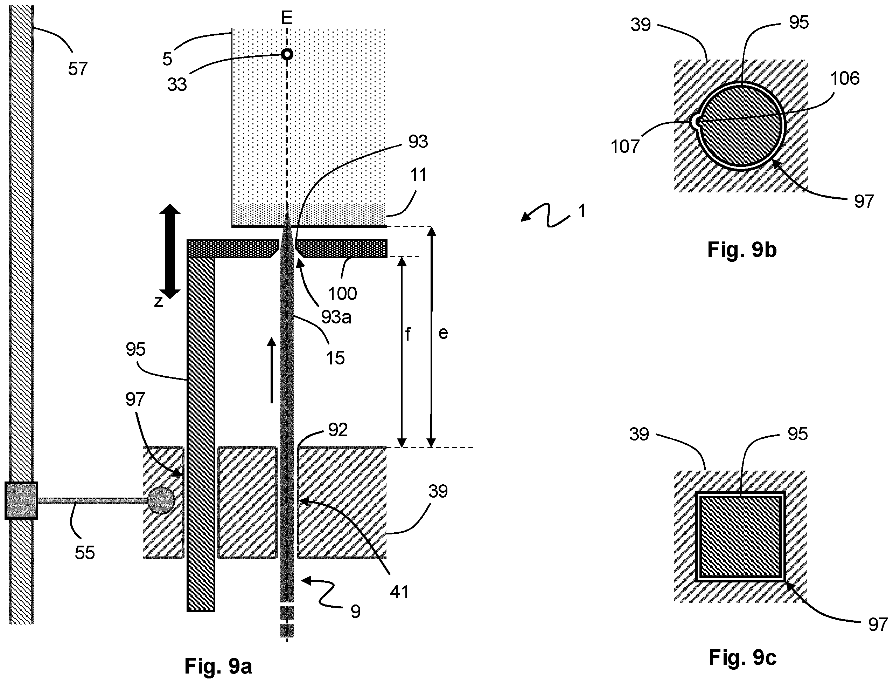

Kennzeichnend für die in Fig. 8 dargestellte prinzipielle Vorgehensweise und ihrem in den Fig. 9a-c gezeigten konkreten Ausführungsbeispiel ist, dass die Bewegungsfreiheit des Lagerpunktelements 100 bezüglich der Platzierungsschablone 39 auf die z-Achse bzw. die Einstichachse E beschränkt ist. Die Verstellkomponente 95 ist hier nicht direkt mit der Referenzfläche 57 verbunden, sondern nur indirekt über die Platzierungsschablone 39, welche mit der Referenzfläche 57 verbunden ist. Es lässt sich also bei der in Fig. 8 und 9a-c dargestellten Vorgehensweise nur der Abstand f für das Lagerpunktelement 100 gegenüber der Platzierungsschablone 39 einstellen, vorzugsweise maximal. Die koaxiale Ausrichtung des Lagerpunkts 93 bezüglich der Führung 41 ist strukturell bzw. konstruktiv festgelegt und braucht nicht mehr durch eine Bedienperson eingestellt zu werden. Das reduziert einerseits den Aufwand für die Bedienperson und eliminiert andererseits potenzielle Fehlerquellen bei der Positionierung des Lagerpunktelements 100 bzw. des Lagerpunktes 93. Im Ausführungsbeispiel der Figuren 9a-c ist die Verstellkomponente 95 als längliches Stabelement ausgeführt, welches in einer Aufnahme 97 der Platzierungsschablone 39 geführt ist. Gemäß Fig. 9b ist die Verstellkomponente 95 gegen eine Verdrehung um die z-Achse durch eine männliche Ausformung 106 gesichert, die in eine entsprechende weibliche Einformung 107 in der Aufnahme 97 der Platzierungsschablone 39 eingreift. Es versteht sich, dass die männliche Ausformung 106 alternativ an der Aufnahme 97 der Platzierungsschablone 39 angeordnet sein kann und die weibliche Einformung 107 entsprechend an der Verstellkomponente 95. In Fig. 9c wird die Verdrehsicherung durch einen nicht-kreisförmigen, hier rechteckigen oder quadratischen Querschnitt der Verstellkomponente 95 erzielt.

Bei der in Fig. 8 dargestellten prinzipiellen Vorgehensweise und der konkreten Ausführungsform der Figuren 9a-c ist es die Platzierungsschablone 39, die über die Befestigungsvorrichtung 55 mit der Referenzfläche 57 verbunden ist, während die Verstellkomponente 95 - im Gegensatz zur zuvor beschriebenen Vorgehensweise - keine direkte Verbindung mehr mit der Referenzfläche 57 aufweist, sondern stattdessen mit der Platzierungsschablone 39 eine direkte und bewegliche Verbindung aufweist. Daraus folgt, dass die Verstellkomponente 95 allein und zwar entlang einer Achse bewegt werden kann, dass sich aber bei einer Bewegung der Platzierungsschablone 39 die Verstellkomponente 95 immer mitbewegt. Dieses Verbindungs- und das daraus resultierende Abhängigkeitsprinzip der verschiedenen Komponenten mit- und zueinander ist sinnvoll, wenn zunächst die Platzierungsschablone 39 in den richtigen bzw. finalen Abstand zum Körper 5 gebracht werden soll (Realisierung des Abstandes d zwischen Platzierungsschablone und virtueller Sollpunktgitterstruktur innerhalb des Körpers 5) und erst im Anschluss daran das Lagerpunktelement 100 bzw. sein oder seine Lagerpunkte 93 in die finale Position, also nahe an die Haut 11, gebracht werden sollen. Für den Fall, dass zuerst das Lagerpunktelement 100 mit seinem bzw. seinen Lagerpunkten 93 in die finale Position, also nahe an der Haut 11, gebracht werden soll und erst im Anschluss daran die finale Platzierung der Platzierungsschablone 39 erfolgen soll, ist ein anderes Verbindungs- und daraus resultierendes Abhängigkeitsverhältnis der verschiedenen Komponenten mit- und zueinander sinnvoll (ohne Abbildung): Die Verstellkomponente 95 wird über eine Befestigungsvorrichtung 55 direkt mit der Referenzfläche 57 verbunden, während die Platzierungsschablone 39 nun keine direkte Verbindung mehr mit der Referenzfläche 57 aufweist, sondern stattdessen über eine Verstellkomponente 95 eine direkte bewegliche Verbindung aufweist. Daraus folgt, dass die Platzierungsschablone 39 allein und zwar entlang einer Achse bewegt werden kann, dass sich aber bei einer Bewegung der Verstellkomponente 95 die Platzierungsschablone 39 immer mitbewegt.

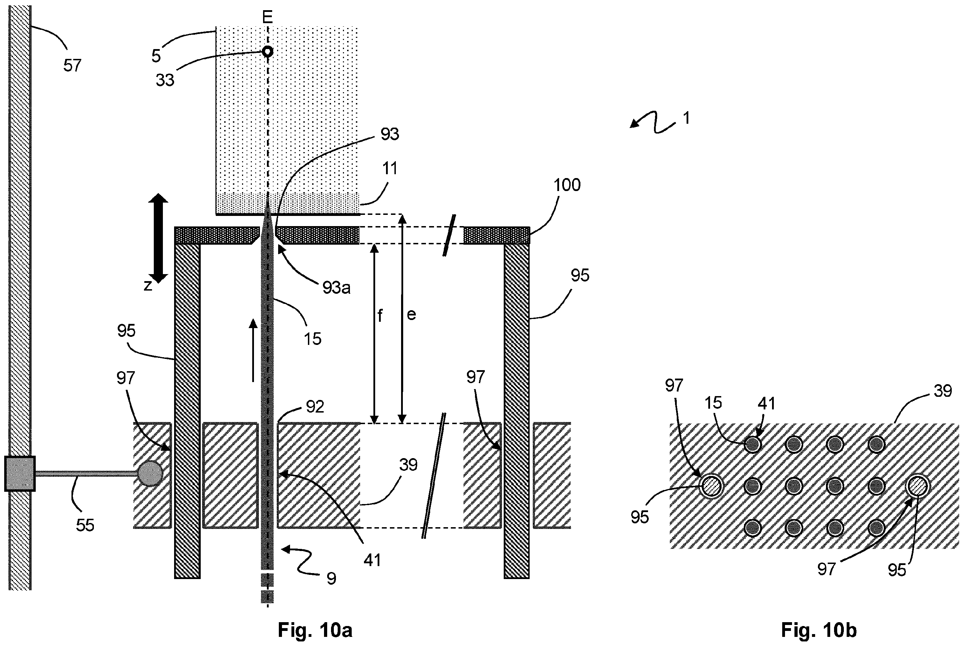

In Fig. 10a,b ist das Lagerpunktelement 100 als Lochplatte mit Löchern 93a ausgeführt, wobei jedes Loch 93a einen Lagerpunkt 93 definiert, wobei die Löcher 93a koaxial zu den Führungen 41 der Platzierungsschablone 39 angeordnet oder anordenbar sind. Die Verstellkomponente 95 ist hier in Form von in Aufnahmen 97 der Platzierungsschablone 39 geführten länglichen Stabelementen ausgeführt, die parallel zueinander in z-Richtung, also der Einstichachse E, verlaufen. Wie in Fig. 8 und 9 lässt sich dadurch das Lagerpunktelement 100 nur in z-Richtung relativ zur Platzierungsschablone 39 positionieren, um einen gewünschten maximalen Abstand f zwischen dem Lagerpunktelement 100 und dem distalen Ende 92 der Führungen 41 der Platzierungsschablone 39 bzw. einen minimalen Abstand zwischen dem Lagerpunktelement 100 und der Haut 11 einzustellen. Wie in Fig. 10b gezeigt, ist in der Platzierungsschablone 39 in einer xy-Ebene quer zur Einstichachse E ein Raster an Führungen 41 zwischen den Aufnahmen 97 angeordnet, durch welche die Lichtapplikatoren 9 mit ihren Einführabschnitten 15 hindurchgeführt werden können.

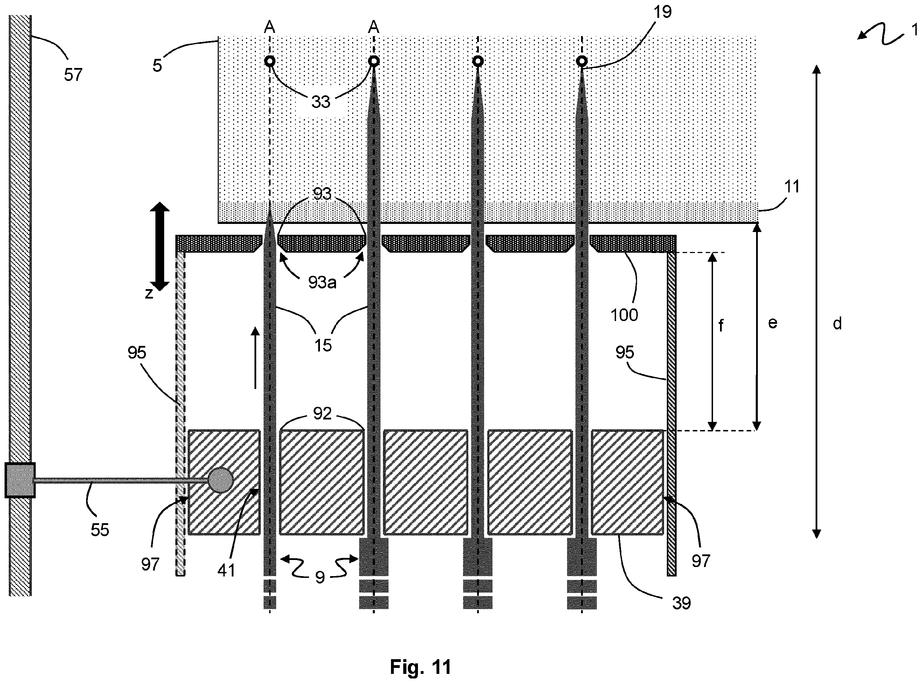

Fig. 11 zeigt eine Ausführungsform des erfindungsgemäßen Systems 1 bei der Durchführung einer transkutanen photodynamischen Therapie (PDT) in einem organischen Körper 5, nachdem der Abstand f des Lagerpunktelements 100 vom distalen Ende 92 der Führungen 41 der Platzierungsschablone 39 eingestellt wurde und die erste drei Lichtapplikatoren 9 bereits mit ihren Applikatorspitzen 19 final an ihren Sollpunkten 33 im Körper 5 platziert wurden. Der vierte Lichtapplikator 9 wird gerade in die Haut 11 eingestochen. Durch das im Abstand f und damit nahe der Haut 11 positionierte Lagerpunktelement 100 ist das Risiko einer Verbiegung der Lichtapplikatoren 9 beim Hauteinstich erheblich reduziert.

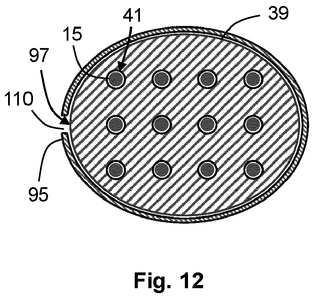

Anders als in den Ausführungsbeispielen gemäß Figuren 10a-c ist die Verstellkomponente 95 hier nicht in Form von in Aufnahmen 97 der Platzierungsschablone 39 geführten länglichen Stabelementen ausgeführt, sondern als rohrförmiges Element, das die Platzierungsschablone 39 umfangseitig umgibt. Eine laterale Außenwandung der Platzierungsschablone 39 bildet dann die Aufnahme 97. In Fig. 12 ist gezeigt, dass eine Verdrehsicherung beispielweise durch einen ellipsenförmigen oder anderen nicht-kreisförmigen Querschnitt sichergestellt werden kann. Ein seitlicher Schlitz 110 in der rohrförmigen Verstellkomponente 95 ist dazu vorgesehen, dass die Befestigungsvorrichtung 55 seitlich hindurchragen kann, um die Platzierungsschablone 39 unabhängig von der Position des Lagerpunktelements 100 gegenüber der Referenzfläche 57 fixieren zu können.

Die in den Figuren 11 und 12 dargestellte Ausführungsform ist so konzipiert, dass zuerst die Platzierungsschablone 39 platziert und fixiert wird, nämlich so, dass sich die Fixierpunktgitterstruktur 37 außerhalb des Körpers 5 im Abstand d zur virtuellen Sollpunktgitterstruktur 35 innerhalb des Körpers 5 befindet, und erst im Anschluss daran die finale Platzierung der Verstellkomponente 95, vorzugsweise nahe der Haut 11, erfolgt. Auch bei dieser Ausführungsform ist die umgekehrte Vorgehensweise denkbar, d.h. dass zunächst die Verstellkomponente 95 final (nahe der Haut 11) platziert wird und erst im Anschluss daran die Platzierungsschablone 39 final platziert wird. Bei dieser modifizierten Vorgehensweise verbindet die Befestigungsvorrichtung 55 aus der Fig. 11 die Referenzfläche 57 dann vorzugsweise mit dem Lagerpunktelement 100, z.B. mit der rohrförmigen Verstellkomponente 95, und nicht mehr mit der Platzierungsschablone 39. Auf den seitlichen Schlitz 110 in der rohrförmigen Verstellkomponente 95 kann dann verzichtet werden. Der Aufbau für diese modifizierte Vorgehensweise ist nicht abgebildet.

### Bezugszeichenliste:

- 1: System
- 3: Organ
- 4: umgebendes, mit dem Organ 3 direkt verbundenes Gewebe
- 5: Körper
- 7: pathologischer Gewebebereich
- 9: Lichtapplikator
- 10: erster Lichtapplikator
- 11: Haut
- 13: distales Ende des Einführabschnitts
- 15: Einführabschnitt des Lichtapplikators
- 17: lichtemittierendes Element / LED
- 19: Applikatorspitze
- 21: Lichtkugel
- 27: Griffelement
- 33: Sollpunkt
- 35: Sollpunktgitterstruktur
- 37: Fixierpunktgitterstruktur
- 39: Platzierungsschablone
- 41: Führung
- 43: Fixierpunkt
- 45: Sollpunktgitterfläche
- 46: Sollpunktgitterfläche
- 47: Fixierpunktgitterfläche
- 51: Fixierpunktaufnahme
- 53: Fixierpunktaufnahmegitterfläche
- 55: Befestigungsvorrichtung
- 57: Referenzfläche
- 59: Eingriffselement
- 61: Eingriffsaufnahme
- 63: Vorsprung
- 65: Aufnahmesicherung
- 67: Eingriffselement
- 69: Eingriffsaufnahme
- 71: erster Schablonenteil
- 73: Untergruppe von Führungen bzw. Lichtapplikatoren
- 75: zweiter Schablonenteil
- 77: Untergruppe von Führungen bzw. Lichtapplikatoren
- 91: Inhomogenität
- 92: distales Ende der Führung der Platzierungsschablone
- 93: Lagerpunkt
- 93a: Öffnung im Lagerpunktelement
- 94: Gelenke
- 95: Verstellkomponente
- 97: Aufnahme
- 100: Lagerpunktelement
- 106: männliche Ausformung
- 107: weibliche Einformung
- 110: Schlitz

## Patentansprüche

1. System (1) zur Durchführung einer transkutanen photodynamischen Therapie (PDT) in einem Organ (3) oder Organsegment eines organischen Körpers (5),
wobei das System (1)
- eine Mehrzahl von Lichtapplikatoren (9),
- eine Versorgungseinheit zum Versorgen der Lichtapplikatoren (9) mit Licht und/oder Strom,
- eine in einer definierten Position zum organischen Körper (5) platzierbare Platzierungsschablone (39) zum definierten Ausrichten, Positionieren und Fixieren der Lichtapplikatoren (9), und
- ein Lagerpunktelement (100) aufweist,
wobei die Lichtapplikatoren (9) jeweils einen nadelartigen Einführabschnitt (15) zum transkutanen Einstechen entlang einer Einstichachse (E) in das Organ (3) oder Organsegment, am distalen Ende (13) des Einführabschnitts (15) eine lichtemittierende Applikatorspitze (19) und mindestens einen definierten Fixierpunkt (43) in einem proximalen Abstand d von der Applikatorspitze (19) aufweisen, wobei der Abstand d für jeden der Lichtapplikatoren (9) identisch ist, wobei das Lagerpunktelement (100) für jeden der Lichtapplikatoren (9) einen mit der Einstichachse (E) koaxial positionierten oder positionierbaren Lagerpunkt (93) zum lateralen Lagern des jeweiligen Lichtapplikators (9) definiert, wobei ein distaler Abstand f des Lagerpunkts (93) von der Platzierungsschablone (39) einstellbar ist.

2. System (1) nach Anspruch 1, wobei der Lagerpunkt (93) durch eine Öffnung (93a) im Lagerpunktelement (100) gebildet ist, durch welche der jeweilige Lichtapplikator (9) passgenau durchsteckbar ist.

3. System (1) nach einem der vorhergehenden Ansprüche, wobei das Lagerpunktelement (100) zumindest entlang der Einstichachse (E) relativ zur Platzierungsschablone (39) beweglich gelagert und positioniert arretierbar ist und/oder die Platzierungsschablone (39) entlang der Einstichachse (E) relativ zum Lagerpunktelement (100) beweglich gelagert und positioniert arretierbar ist.

4. System (1) nach einem der vorhergehenden Ansprüche, wobei das Lagerpunktelement (100) derart geführt gelagert mit der Platzierungsschablone (39) gekoppelt ist, dass das Lagerpunktelement (100) relativ zur Platzierungsschablone (39) ausschließlich linear entlang der Einstichachse (E) beweglich ist und/oder die Platzierungsschablone (39) derart geführt gelagert mit dem Lagerpunktelement (100) gekoppelt ist, dass die Platzierungsschablone (39) relativ zum Lagerpunktelement (100) ausschließlich linear entlang der Einstichachse (E) beweglich ist.

5. System (1) nach einem der vorhergehenden Ansprüche, wobei das Lagerpunktelement (100) als Lochplatte mit Löchern (93a) ausgeführt ist, wobei jedes Loch (93a) einen Lagerpunkt (93) definiert, wobei die Löcher (93a) koaxial zu Lichtapplikatoraufnahmen der Platzierungsschablone (39) angeordnet oder anordenbar sind.

6. System (1) nach einem der vorhergehenden Ansprüche, wobei das Lagerpunktelement (100) eine von der Platzierungsschablone (39) abgewandte Hautauflagefläche aufweist, wobei die Hautauflagefläche auf die Haut eines organischen Körpers (5) auflegbar und dort positioniert fixierbar ist.

7. System (1) nach einem der vorhergehenden Ansprüche, wobei das Lagerpunktelement (100) auch in einer Ebene quer zur Einstichachse (E) relativ zur Platzierungsschablone (39) positionierbar beweglich ist und/oder die Platzierungsschablone (39) auch in einer Ebene quer zur Einstichachse (E) relativ zum Lagerpunktelement (100) positionierbar beweglich ist.

8. System (1) nach einem der vorhergehenden Ansprüche, wobei die Lichtapplikatoren (9) unabhängig voneinander einzeln in die Platzierungsschablone (39) und das Lagerpunktelement (100) einsetzbar sind.

9. System (1) nach einem der vorhergehenden Ansprüche, wobei die Platzierungsschablone (39) mindestens einen ersten Schablonenteil (71) aufweist, der eine erste Untergruppe (73) der Fixierpunktaufnahmen (51) definiert, und mindestens einen zweiten Schablonenteil (75) aufweist, der eine zweite Untergruppe (77) der Fixierpunktaufnahmen (51) definiert, wobei der erste Schablonenteil (71) relativ zum zweiten Schablonenteil (75) entlang der Einstichachse (E) geführt verschiebbar ist.

10. System (1) nach Anspruch 9, wobei die erste Untergruppe (73) der Fixierpunktaufnahmen (51) ein erstes Fixierpunktaufnahmeteilgitter und die zweite Untergruppe (77) der Fixierpunktaufnahmen (51) ein zweites Fixierpunktaufnahmeteilgitter definiert, wobei das zweite Fixierpunktaufnahmeteilgitter quer zur Einstichachse (E) um einen lateralen Gitterversatz versetzt zum ersten Fixierpunktaufnahmeteilgitter ist.

11. System (1) nach einem der Ansprüche 9 oder 10, wobei die erste Untergruppe (73) der Fixierpunktaufnahmen (51), die zweite Untergruppe (77) der Fixierpunktaufnahmen (51) sowie die Lichtapplikatoren (9) zusammen eine dreidimensionale Fixierpunktgitterstruktur (37) definieren, wobei die Fixierpunktgitterstruktur (37) einer virtuellen organspezifischen Sollpunktgitterstruktur (35) für die lichtemittierenden Applikatorspitzen (19) im Organ (3) oder Organsegment entspricht und um einen Abstand d entlang der Einstichachse (E) parallelverschoben zur Sollpunktgitterstruktur (35) ist, wobei jeder Lichtapplikator (9) mindestens einen definierten Fixierpunkt (43) im Abstand d von der lichtemittierenden Applikatorspitze (19) aufweist.

12. System (1) nach einem der Ansprüche 9 bis 11, wobei der zweite Schablonenteil (75) in proximale Richtung relativ zum ersten Schablonenteil (71) nur bis zur Anlage an den ersten Schablonenteil (71) verschiebbar ist, wobei bei Anlage des zweiten Schablonenteils (75) am ersten Schablonenteil (71) die vom ersten Schablonenteil (71) definierte erste Untergruppe (73) der Fixierpunktaufnahmen (51) distal von der vom zweiten Schablonenteil (75) definierten zweiten Untergruppe (77) der Fixierpunktaufnahmen (51) liegt.

13. System (1) nach einem der Ansprüche 9 bis 12, wobei der erste Schablonenteil (71) eine erste Untergruppe der Fixierpunktaufnahmen (51) einer ersten Fixierpunktaufnahmegitterfläche (53a) und der zweite Schablonenteil (75) eine zweite Untergruppe der Fixierpunktaufnahmen (51) einer zweiten Fixierpunktaufnahmegitterfläche (53b) aufweist.

14. System (1) nach einem der vorhergehenden Ansprüche, wobei die Versorgungseinheit dazu eingerichtet ist, die Lichtapplikatoren (9) mit Strom zu versorgen und jeder Lichtapplikator (9) am distalen Ende (13) des Einführabschnitts (15) eine mit dem Strom betreibbare LED (17) aufweist.

15. System (1) nach einem der vorhergehenden Ansprüche, wobei die Lichtapplikatoren (9) jeweils mindestens einen Fixierpunkt (43) aufweisen, der durch einen Anschlag ausgebildet und arretierbar ist.
